# FASCICULE DE BREVET EUROPEEN

(11) **EP 2 922 969 B1**
(45) Date de publication et mention de la délivrance du brevet: **27.03.2019**
(21) Numéro de dépôt: 13795763.5
(22) Date de dépôt: 26.11.2013
(51) Int. Cl.: C12Q 1/68

(54) **MARQUEURS MOLÉCULAIRES ET MÉTHODES POUR L'IDENTIFICATION DES GÉNOTYPES DE PALMIER DATTIER**
MOLEKULARE MARKER UND VERFAHREN ZUR IDENTIFIZIERUNG VON DATTELPALMENGENOTYPEN
MOLECULAR MARKERS AND METHODS FOR IDENTIFYING DATE PALM GENOTYPES

(30) Priorité: 26.11.2012 FR 1261226
(43) Date de publication de la demande: 30.09.2015
(73) Titulaire: Institut De Recherche Pour Le Développement (IRD), 13002 Marseille 2 (FR); Laboratoire de Genetique Moleculaire, 2092 Tunis (TN)
(72) Inventeur: ABERLENC-BERTOSSI, Frédérique, F-34170 Castelnau-le-Lez (FR); PINTAUD, Jean-Christophe, F-34790 Grabels (FR); CHABRILLANGE, Nathalie, F-34790 Grabels (FR); CHERIF, Emira, Ariana 2080 (TN); CASTILLO-PEREZ, Karina, F-34170 Castelnau-le-Lez (FR); ZEHDI, Salwa, 2094-Mnihla (TN)
(74) Mandataire: Cabinet Plasseraud
(86) Numéro de dépôt international: PCT/EP2013/074728
(87) Numéro de publication internationale: WO 2014/080034

(56) Documents cités:
- HENDERSON S A ET AL: "Genetic isolation of Cape Verde Island Phoenix atlantica (Arecaceae) revealed by microsatellite markers", CONSERVATION GENETICS, KLUWER ACADEMIC PUBLISHERS, DO, vol. 7, no. 2, 22 février 2006 (2006-02-22), pages 213-223, XP019393502, ISSN: 1572-9737, DOI: 10.1007/S10592-006-9128-7 cité dans la demande
- BILLOTTE N ET AL: "Nuclear microsatellite markers for the date palm (Phoenix dactylifera L.): characterization and utility across the genus Phoenix and in other palm genera", MOLECULAR ECOLOGY NOTES, BLACKWELL SCIENCE, OXFORD, GB, vol. 4, no. 2, 1 janvier 2004 (2004-01-01) , pages 256-258, XP002487759, ISSN: 1471-8278, DOI: 10.1111/J.1471-8286.2004.00634.X cité dans la demande
- KHALED ELMEER ET AL: "New microsatellite markers for assessment of genetic diversity in date palm (Phoenix dactylifera L.)", 3 BIOTECH, vol. 1, no. 2, 27 mai 2011 (2011-05-27), pages 91-97, XP055067021, ISSN: 2190-572X, DOI: 10.1007/s13205-011-0010-z
- AKKAK A ET AL: "Development and evaluation of microsatellite markers in Phoenix dactylifera L. and their transferability to other Phoenix species", BIOLOGIA PLANTARUM, KLUWER ACADEMIC PUBLISHERS, DO, vol. 53, no. 1, 21 mars 2009 (2009-03-21), pages 164-166, XP019670821, ISSN: 1573-8264
- HESAM ARABNEZHAD ET AL: "Development, characterization and use of microsatellite markers for germplasm analysis in date palm (L.)", SCIENTIA HORTICULTURAE, ELSEVIER SCIENCE PUBLISHERS, XX, vol. 134, 28 novembre 2011 (2011-11-28), pages 150-156, XP028437868, ISSN: 0304-4238, DOI: 10.1016/J.SCIENTA.2011.11.032 [extrait le 2011-12-06]
- EMAN K AL-DOUS ET AL: "De novo genome sequencing and comparative genomics of date palm (Phoenix dactylifera)", NATURE BIOTECHNOLOGY, vol. 29, no. 6, 1 juin 2011 (2011-06-01), pages 521-527, XP055067015, ISSN: 1087-0156, DOI: 10.1038/nbt.1860 cité dans la demande

## Description

### Domaine de l'Invention

La présente invention concerne deux jeux de marqueurs moléculaires spécifiques de la variété génétique du palmier dattier pour identifier les génotypes et les cultivars de palmier dattier.

### Contexte de l'Invention

Le palmier dattier ou dattier (*Phoenix dactylifera L*.) est une plante monocotylédone de la famille des Arécacées largement cultivée pour ses fruits, les dattes. Dans les zones arides, le dattier joue non seulement un rôle économique majeur, grâce à la production des dattes qui constituent la base de l'alimentation humaine et animale, mais également un rôle écologique puisqu'il confère sa structure à l'oasis. Le palmier dattier est cultivé par l'homme depuis des millénaires ; les premiers écrits et dessins confirmant sa culture remontant à 6000 ans avant notre ère. Adapté à la culture dans les zones arides et semi-arides, il a progressivement été introduit sur les cinq continents.

La culture du palmier dattier a évolué d'un système traditionnel, riche et diversifié vers un système industriel centré sur une oligoculture monovariétale. Ceci constitue un risque potentiel d'érosion génétique du patrimoine. La perte de diversité génétique est aggravée par les stress biotiques (maladies et ravageurs) et abiotiques (sécheresse et salinité des sols) auxquels sont soumises les palmeraies. C'est pourquoi il devient crucial, pour de nombreux pays producteurs, de caractériser la diversité des ressources génétiques, afin d'organiser leur conservation et leur sauvegarde.

La caractérisation de la diversité des cultivars de palmier dattier peut être obtenue par l'utilisation de marqueurs morphologiques ou de marqueurs moléculaires de type RAPD (Random Amplified Polymorphic DNA), RFLP (Restriction Fragment Length Polymorphism), AFLP (Amplified Fragment Length Polymorphism) et SSR (Simple Sequence Repeat). Au cours de la dernière décennie, le développement et l'utilisation de marqueurs moléculaires a permis d'améliorer l'étude des ressources génétiques dans les organismes végétaux en réduisant l'incertitude due à la variation des caractères morphologiques et agronomiques sous l'effet des facteurs environnementaux.

Les marqueurs microsatellites ou SSR sont particulièrement intéressants : ils sont abondants dans le génome ; ils possèdent des niveaux élevés de polymorphisme, ce qui procure une grande fiabilité dans la caractérisation des individus ; et ils sont co-dominants et transmis selon la loi mendélienne.

Chez le palmier dattier, 16 marqueurs microsatellites dinucléotidiques, (GA)ₙ, ont été générés à partir d'une banque d'ADN (Billotte et al., Molecular Ecology Notes, 2004, 4: 256-258). Ces marqueurs ont été validés pour leur précision dans la distinction des différents cultivars de Tunisie (Zehdi et al., Hereditas, 2004, 141: 278-287) et du Soudan (Elshibli et al., Genetica, 2008, 134: 251-260). Cependant, la majorité des études de diversité du palmier dattier ont été réalisées à l'aide de différents jeux de marqueurs. De plus, les marqueurs microsatellites ont généralement été validés sur des échantillonnages réalisés au niveau géographique local. Il s'ensuit que les résultats obtenus par les différentes équipes ne sont pas compatibles au niveau international. En outre, certains marqueurs dinucléotidiques sont difficiles à analyser en raison de la taille des allèles générés, variant parfois d'un seul nucléotide. Depuis 2011, le séquençage partiel du génome du palmier dattier, qui a récemment été publié (Al-Dous et al., Nature Biotechnology, 2011, 29: 521-527), offre la possibilité d'accéder à un grand nombre de marqueurs avec divers motifs répétés.

Il est donc important de développer de nouvelles stratégies, validées sur des échantillonnages représentant la diversité mondiale de l'espèce, permettant la normalisation des procédures d'identification dans l'objectif de caractériser l'agrodiversité du palmier dattier. De telles stratégies ouvriraient de nouvelles perspectives pour réintroduire de la biodiversité dans les palmeraies et mettre en oeuvre des programmes d'amélioration génétique. Il existe également un besoin d'outils permettant la certification de plants propagés végétativement dont sont demandeurs les cultivateurs dans les palmeraies et les laboratoires de production de vitroplants.

### Résumé de l'Invention

D'une façon générale, il est décrit ici des jeux de marqueurs moléculaires optimisés, composés d'un nombre limité de marqueurs sélectionnés pour leur précision et leur capacité à générer un polymorphisme élevé. Ces jeux de marqueurs ont été validés sur une collection de génotypes représentant la diversité mondiale du palmier dattier. Plus spécifiquement, le premier jeu comprend 19 marqueurs mini- et micro-satellites et permet d'analyser 100% des génotypes. Ce jeu est particulièrement utile pour les recherches sur l'analyse de la diversité génétique, l'identification des génotypes, l'analyse d'hybrides et des flux de gènes au sein de *Phoenix.* Le deuxième jeu comprend 7 marqueurs mini- et micro-satellites et permet d'identifier 99% des génotypes. Ce deuxième jeu est particulièrement utile pour la certification des cultivars. Les inventeurs ont également conçu une bibliothèque de cultivars de référence couvrant la diversité mondiale du palmier dattier et une base de données qui gère l'ensemble des informations générées.

En conséquence, dans un premier aspect, la présente invention concerne une méthode d'identification du génotype d'un palmier dattier par détection, à l'aide d'un jeu de marqueurs moléculaires mini-satellites et micro-satellites, d'un polymorphisme de type single sequence repeat (SSR) dans le génome du palmier dattier, caractérisée en ce que la détection du polymorphisme SSR comprend pour chaque marqueur moléculaire du jeu :
- l'amplification, à l'aide d'une paire d'amorces spécifiques du marqueur, d'une portion de l'ADN génomique du palmier dattier testé comprenant le mini-satellite ou micro-satellite pour obtenir des amplicons ; et
- l'analyse des amplicons obtenus pour déterminer le génotype du palmier dattier testé,
et caractérisée en ce que le jeu consiste en les 19 marqueurs moléculaires: mPdCIR010, mPdCIR015, mPdCIR016, mPdCIR025, mPdCIR032, mPdCIR035, mPdCIR057, mPdCIR063, mPdCIR078, mPdCIR085, CpfM12, PdCUC3-ssr1, PdCUC3-ssr2, PdAG1-ssr1, PdAP3-ssr, mPdIRD13, mPdIRD031, mPdIRD033, et mPdIRD40, ou en ce que le jeu consiste en les 7 marqueurs moléculaires: CpfM12, mPdIRD031, mPdIRD033, mPdIRD040, PdCUC3-ssr2, PdAP3-ssr, et mPdCIR078, où
- le marqueur mPdCIR010 a, dans le sens 5'→3', un motif (ga) ; est présent dans la séquence de numéro d'ordre GenBank AJ571673.1 ; et est flanqué, en 5', par la séquence nucléotidique SEQ ID NO: 1, et en 3', par la séquence nucléotidique SEQ ID NO: 2 ;
- le marqueur mPdCIR015 a, dans le sens 5'→3', un motif (ga) ; est présent dans la séquence de numéro d'ordre GenBank AJ571674.1 ; et est flanqué, en 5', par la séquence nucléotidique SEQ ID NO: 3, et en 3', par la séquence nucléotidique SEQ ID NO: 4 ;
- le marqueur mPdCIR016 a, dans le sens 5'→3', un motif (ga) ; est présent dans la séquence de numéro d'ordre GenBank AJ571675.1 ; et est flanqué, en 5', par la séquence nucléotidique SEQ ID NO: 5, et en 3', par la séquence nucléotidique SEQ ID NO: 6 ;
- le marqueur mPdCIR025 a, dans le sens 5'→3', un motif (ga) ; est présent dans la séquence de numéro d'ordre GenBank AJ571676.1 ; et est flanqué, en 5', par la séquence nucléotidique SEQ ID NO: 7, et en 3', par la séquence nucléotidique SEQ ID NO: 8 ;
- le marqueur mPdCIR032 a, dans le sens 5'→3', un motif (ga) ; est présent dans la séquence de numéro d'ordre GenBank AJ571677.1 ; et est flanqué, en 5', par la séquence nucléotidique SEQ ID NO: 9, et en 3', par la séquence nucléotidique SEQ ID NO: 10 ;
- le marqueur mPdCIR035 a, dans le sens 5'→3', un motif (ga) ; est présent dans la séquence de numéro d'ordre GenBank AJ571678.1 ; et est flanqué, en 5', par la séquence nucléotidique SEQ ID NO: 11, et en 3', par la séquence nucléotidique SEQ ID NO: 12 ;
- le marqueur mPdCIR057 a, dans le sens 5'→3', un motif (ga) ; est présent dans la séquence de numéro d'ordre GenBank AJ571682.1 ; et est flanqué, en 5', par la séquence nucléotidique SEQ ID NO: 13, et en 3', par la séquence nucléotidique SEQ ID NO: 14 ;
- le marqueur mPdCIR063 a, dans le sens 5'→3', un motif (ga) ; est présent dans la séquence de numéro d'ordre GenBank AJ571683.1 ; et est flanqué, en 5', par la séquence nucléotidique SEQ ID NO: 15, et en 3', par la séquence nucléotidique SEQ ID NO: 16 ;
- le marqueur mPdCIR078 a, dans le sens 5'→3', un motif (ga) ; est présent dans la séquence de numéro d'ordre GenBank AJ571685.1 ; et est flanqué, en 5', par la séquence nucléotidique SEQ ID NO: 17, et en 3', par la séquence nucléotidique SEQ ID NO: 18 ;
- le marqueur mPdCIR085 a, dans le sens 5'→3', un motif (ga) ; est présent dans la séquence de numéro d'ordre GenBank AJ571686.1 ; et est flanqué, en 5', par la séquence nucléotidique SEQ ID NO: 19, et en 3', par la séquence nucléotidique SEQ ID NO: 20 ;
- le marqueur CpfM12 a, dans le sens 5'→3', un motif (ctaactactata) ; est présent dans la séquence de numéro d'ordre GenBank EU043484.1 ; et est flanqué, en en 5', par la séquence nucléotidique SEQ ID NO: 21, et en 3', par la séquence nucléotidique SEQ ID NO: 22 ;
- le marqueur PdCUC3-ssr a, dans le sens 5'→3', un motif (gt+ga) ; est présent dans la séquence de numéro d'ordre GenBank HM622273.1 ; et est flanqué, en en 5', par la séquence nucléotidique SEQ ID NO: 23, et en 3', par la séquence nucléotidique SEQ ID NO: 24 ;
- le marqueur PdCUC3-ssr2 a, dans le sens 5'→3', un motif (ga) ; est présent dans la séquence de numéro d'ordre GenBank HM622273.1 ; et est flanqué, en en 5', par la séquence nucléotidique SEQ ID NO: 25, et en 3', par la séquence nucléotidique SEQ ID NO: 26 ;
- le marqueur PdAG1-ssr1 a, dans le sens 5'→3', un motif (ga) ; est présent dans la séquence de numéro d'ordre GenBank KC188338.1 ; et est flanqué, en en 5', par la séquence nucléotidique SEQ ID NO: 27, et en 3', par la séquence nucléotidique SEQ ID NO: 28 ;
- le marqueur PdAP3-ssr a, dans le sens 5'→3', un motif (ga) ; est présent dans la séquence de numéro d'ordre GenBank KC188337.1 ; et est flanqué, en en 5', par la séquence nucléotidique SEQ ID NO: 29, et en 3', par la séquence nucléotidique SEQ ID NO: 30 ;
- le marqueur mPdIRD13 a, dans le sens 5'→3', un motif (cac) ; est présent dans le scaffold PDK_20s1496731g002_1 de numéro d'ordre GenBank GL741615.1 ; et est flanqué, en en 5', par la séquence nucléotidique SEQ ID NO: 31, et en 3', par la séquence nucléotidique SEQ ID NO: 32 ;
- le marqueur mPdIRD31 a, dans le sens 5'→3', un motif (cca) ; est présent dans le scaffold PDK_20s1419261g003_1 ; et est flanqué, en en 5', par la séquence nucléotidique SEQ ID NO: 33, et en 3', par la séquence nucléotidique SEQ ID NO: 34 ;
- le marqueur mPdIRD33 a, dans le sens 5'→3', un motif (cag) ; est présent dans le scaffold PDK_20s1569281g001_1 ; et est flanqué, en en 5', par la séquence nucléotidique SEQ ID NO: 35, et en 3', par la séquence nucléotidique SEQ ID NO: 36 ; et
- le marqueur mPdIRD40 a, dans le sens 5'→3', un motif (ccagtg) ; est présent dans le scaffold PDK_20s1327401g002_1 ; et est flanqué, en 5', par la séquence nucléotidique SEQ ID NO: 37, et en 3', par la séquence nucléotidique SEQ ID NO: 38.

Dans certains modes de réalisation, la méthode d'identification du génotype d'un palmier dattier est caractérisée en ce que le jeu consiste en les 19 marqueurs moléculaires et les amplifications sont réalisées avec chacune des paires d'amorces spécifiques suivantes :
- une amorce SSR forward de séquence SEQ ID NO: 1 et une amorce SSR reverse de séquence SEQ ID NO: 2 ;
- une amorce SSR forward de séquence SEQ ID NO: 3 et une amorce SSR reverse de séquence SEQ ID NO: 4 ;
- une amorce SSR forward de séquence SEQ ID NO: 5 et une amorce SSR reverse de séquence SEQ ID NO: 6 ;
- une amorce SSR forward de séquence SEQ ID NO: 7 et une amorce SSR reverse de séquence SEQ ID NO: 8 ;
- une amorce SSR forward de séquence SEQ ID NO: 9 et une amorce SSR reverse de séquence SEQ ID NO: 10;
- une amorce SSR forward de séquence SEQ ID NO: 11 et une amorce SSR reverse de séquence SEQ ID NO: 12 ;
- une amorce SSR forward de séquence SEQ ID NO: 13 et une amorce SSR reverse de séquence SEQ ID NO: 14 ;
- une amorce SSR forward de séquence SEQ ID NO: 15 et une amorce SSR reverse de séquence SEQ ID NO: 16 ;
- une amorce SSR forward de séquence SEQ ID NO: 17 et une amorce SSR reverse de séquence SEQ ID NO: 18 ;
- une amorce SSR forward de séquence SEQ ID NO: 19 et une amorce SSR reverse de séquence SEQ ID NO: 20 ;
- une amorce SSR forward de séquence SEQ ID NO: 21 et une amorce SSR reverse de séquence SEQ ID NO: 22 ;
- une amorce SSR forward de séquence SEQ ID NO: 23 et une amorce SSR reverse de séquence SEQ ID NO: 24 ;
- une amorce SSR forward de séquence SEQ ID NO: 25 et une amorce SSR reverse de séquence SEQ ID NO: 26 ;
- une amorce SSR forward de séquence SEQ ID NO: 27 et une amorce SSR reverse de séquence SEQ ID NO: 28 ;
- une amorce SSR forward de séquence SEQ ID NO: 29 et une amorce SSR reverse de séquence SEQ ID NO: 30 ;
- une amorce SSR forward de séquence SEQ ID NO: 31 et une amorce SSR reverse de séquence SEQ ID NO: 32 ;
- une amorce SSR forward de séquence SEQ ID NO: 33 et une amorce SSR reverse de séquence SEQ ID NO: 34 ;
- une amorce SSR forward de séquence SEQ ID NO: 35 et une amorce SSR reverse de séquence SEQ ID NO: 36 ; et
- une amorce SSR forward de séquence SEQ ID NO: 37 et une amorce SSR reverse de séquence SEQ ID NO: 38.

Dans d'autres modes de réalisation, la méthode d'identification du génotype d'un palmier dattier est caractérisée en ce que le jeu consiste en les 7 marqueurs moléculaires et les amplifications sont réalisées avec chacune des paires d'amorces spécifiques suivantes :
- une amorce SSR forward de séquence SEQ ID NO: 17 et une amorce SSR reverse de séquence SEQ ID NO:18 ;
- une amorce SSR forward de séquence SEQ ID NO: 21 et une amorce SSR reverse de séquence SEQ ID NO:22 ;
- une amorce SSR forward de séquence SEQ ID NO: 25 et une amorce SSR reverse de séquence SEQ ID NO: 26 ;
- une amorce SSR forward de séquence SEQ ID NO: 29 et une amorce SSR reverse de séquence SEQ ID NO: 30 ;
- une amorce SSR forward de séquence SEQ ID NO: 33 et une amorce SSR reverse de séquence SEQ ID NO: 34 ;
- une amorce SSR forward de séquence SEQ ID NO: 35 et une amorce SSR reverse de séquence SEQ ID NO: 36 ; et
- une amorce SSR forward de séquence SEQ ID NO: 37 et une amorce SSR reverse de séquence SEQ ID NO: 38.

Dans certains modes de réalisation, la méthode d'identification du génotype d'un palmier dattier est caractérisée en ce que le jeu consiste en les 7 marqueurs moléculaires et l'identification du génotype du palmier dattier testé comprend l'identification ou la certification du cultivar du palmier dattier testé.

Dans certains modes de réalisation, la méthode d'identification du génotype d'un palmier dattier est caractérisée en ce que l'analyse des amplicons obtenus pour déterminer le génotype du palmier dattier testé comprend, pour chacun des marqueurs moléculaires :
- la séparation, en fonction de leur taille, des amplicons obtenus par amplification d'une portion de l'ADN génomique du palmier dattier testé, et
- la comparaison des tailles desdits amplicons avec les tailles des amplicons obtenus par amplification, dans les mêmes conditions, d'une portion de l'ADN génomique de palmiers dattiers contrôles.

Dans certains modes de réalisation, la méthode d'identification du génotype d'un palmier dattier est caractérisée en ce qu'elle est réalisée sur de l'ADN génomique extrait d'un échantillon du palmier dattier testé, où l'échantillon du palmier dattier est un échantillon de protoplaste, de cal, d'embryons, de feuille, de stipe, de racine, de rejets ou de bouture du palmier dattier.

Dans un autre aspect, la présente invention concerne un jeu de 19 paires d'amorces spécifiques de marqueurs moléculaires mini-satellites et micro-satellites pour identifier le génotype d'un palmier dattier, le jeu étant constitué des paires d'amorces suivantes :
- une amorce SSR forward de séquence SEQ ID NO: 1 et une amorce SSR reverse de séquence SEQ ID NO: 2 ;
- une amorce SSR forward de séquence SEQ ID NO: 3 et une amorce SSR reverse de séquence SEQ ID NO: 4 ;
- une amorce SSR forward de séquence SEQ ID NO: 5 et une amorce SSR reverse de séquence SEQ ID NO: 6 ;
- une amorce SSR forward de séquence SEQ ID NO: 7 et une amorce SSR reverse de séquence SEQ ID NO: 8 ;
- une amorce SSR forward de séquence SEQ ID NO: 9 et une amorce SSR reverse de séquence SEQ ID NO: 10;
- une amorce SSR forward de séquence SEQ ID NO: 11 et une amorce SSR reverse de séquence SEQ ID NO: 12 ;
- une amorce SSR forward de séquence SEQ ID NO: 13 et une amorce SSR reverse de séquence SEQ ID NO: 14 ;
- une amorce SSR forward de séquence SEQ ID NO: 15 et une amorce SSR reverse de séquence SEQ ID NO: 16 ;
- une amorce SSR forward de séquence SEQ ID NO: 17 et une amorce SSR reverse de séquence SEQ ID NO: 18 ;
- une amorce SSR forward de séquence SEQ ID NO: 19 et une amorce SSR reverse de séquence SEQ ID NO: 20 ;
- une amorce SSR forward de séquence SEQ ID NO: 21 et une amorce reverse de séquence SEQ ID NO: 22 ;
- une amorce SSR forward de séquence SEQ ID NO: 23 et une amorce SSR reverse de séquence SEQ ID NO: 24 ;
- une amorce SSR forward de séquence SEQ ID NO: 25 et une amorce SSR reverse de séquence SEQ ID NO: 26 ;
- une amorce SSR forward de séquence SEQ ID NO: 27 et une amorce SSR reverse de séquence SEQ ID NO: 28 ;
- une amorce SSR forward de séquence SEQ ID NO: 29 et une amorce SSR reverse de séquence SEQ ID NO: 30 ;
- une amorce SSR forward de séquence SEQ ID NO: 31 et une amorce SSR reverse de séquence SEQ ID NO: 32 ;
- une amorce SSR forward de séquence SEQ ID NO: 33 et une amorce SSR reverse de séquence SEQ ID NO: 34 ;
- une amorce SSR forward de séquence SEQ ID NO: 35 et une amorce SSR reverse de séquence SEQ ID NO: 36 ; et
- une amorce SSR forward de séquence SEQ ID NO: 37 et une amorce SSR reverse de séquence SEQ ID NO: 38

Dans le même aspect, la présente invention concerne également un jeu de 7 paires d'amorces spécifiques de marqueurs moléculaires mini-satellites et micro-satellites pour identifier ou certifier le cultivar d'un palmier dattier testé, le jeu étant constitué des paires d'amorces suivantes :
- une amorce SSR forward de séquence SEQ ID NO: 17 et une amorce SSR reverse de séquence SEQ ID NO:18 ;
- une amorce SSR forward de séquence SEQ ID NO: 21 et une amorce SSR reverse de séquence SEQ ID NO:22 ;
- une amorce SSR forward de séquence SEQ ID NO: 25 et une amorce SSR reverse de séquence SEQ ID NO: 26 ;
- une amorce SSR forward de séquence SEQ ID NO: 29 et une amorce SSR reverse de séquence SEQ ID NO: 30 ;
- une amorce SSR forward de séquence SEQ ID NO: 33 et une amorce SSR reverse de séquence SEQ ID NO: 34 ;
- une amorce SSR forward de séquence SEQ ID NO: 35 et une amorce SSR reverse de séquence SEQ ID NO: 36 ; et
- une amorce SSR forward de séquence SEQ ID NO: 37 et une amorce SSR reverse de séquence SEQ ID NO: 38.

Dans certains modes de réalisation, le jeu de 19 ou de 7 paires d'amorces selon l'invention, est caractérisé en ce qu'une des amorces de chacune des paires d'amorces comprend un marqueur détectable.

Dans un autre aspect, l'invention concerne l'utilisation du jeu de 19 paires d'amorces pour l'identification d'un palmier dattier, et également l'utilisation du jeu de 7 paires d'amorces pour l'identification ou la certification du cultivar d'un palmier dattier.

Dans encore un autre aspect, l'invention concerne un kit pour l'identification du génotype de palmiers dattiers comprenant le jeu de 19 paires d'amorces et des instructions pour identifier le génotype par une méthode d'identification du génotype d'un palmier dattier, où le jeu de marqueurs moléculaires mini-satellites et micro-satellites consiste en les 19 marqueurs moléculaires.

L'invention concerne aussi un kit pour l'identification ou la certification du cultivar de palmiers dattiers comprenant le jeu de 7 paires d'amorces et des instructions pour identifier le génotype par une méthode d'identification du génotype d'un palmier dattier, où le jeu de marqueurs moléculaires mini-satellites et micro-satellites consiste en les 7 marqueurs moléculaires.

Une description plus détaillée de certains modes de réalisation préférés de l'invention est donnée ci-dessous.

### Description Détaillée de l'Invention

Comme mentionné ci-dessus, deux jeux de marqueurs moléculaires sont décrits ici pour l'étude de la biodiversité génétique du palmier dattier et l'identification des cultivars.

### I - Jeux de Marqueurs de Génotypage du Palmier Dattier

Les inventeurs ont assemblé un nouvel ensemble optimisé de mini- et micro-satellites défini par l'exploitation de marqueurs microsatellites décrits dans la littérature ou identifiés à partir des ressources génomiques disponibles.

On entend ici par "minisatellite", une séquence d'ADN formée par une répétition continue de motifs composés de 10 à 100 nucléotides et le plus souvent flanquée par des régions conservées. On entend ici par "microsatellite", une séquence d'ADN formée par une répétition continue de motifs composés de 1 à 10 nucléotides et le plus souvent flanquée par des régions conservées. D'une manière générale, la longueur de ces séquences SSR (minisatellite ou microsatellite), c'est-à-dire le nombre de répétitions du motif, est variable d'une espèce à l'autre, d'un individu à l'autre et d'un allèle à l'autre chez un même individu. L'ensemble des informations relatives aux longueurs de séquences des marqueurs mini- et micro-satellites sont indicatives du génotype du palmier dattier.

Le premier jeu de marqueurs comprend 19 marqueurs SSR : CpfM12, mPdIRD031, mPdIRD033, mPdIRD040, PdCUC3-ssr2, PdAP3-ssr, mPdCIR078, mPdCIR015, mPdCIR016, mPdCIR032, mPdCIR035, mPdCIR057, mPdCIR085, PdAG1-ssr, mPdCIR010, mPdCIR025, mPdCIR063, PdCUC3-ssr1, et mPdIRD013.

Le second jeu de marqueurs comprend 7 marqueurs SSR : CpfM12, mPdIRD031, mPdIRD033, mPdIRD040, PdCUC3-ssr2, PdAP3-ssr, et mPdCIR078.

Les marqueurs mPdCIR078, mPdCIR015, mPdCIR016, mPdCIR032, mPdCIR035, mPdCIR057, mPdCIR085, mPdCIR010, mPdCIR025, mPdCIR063 sont des loci dinucléotidiques (GA)n, qui ont été décrits par Billotte et al. (Molecular Ecology Notes, 2004, 4: 256-258).

Les marqueurs mPdIRD031, mPdIRD033, mPdIRD040 et mPdIRD013 sont des répétitions en tandem microsatellites de 3 ou 6 paires de bases que les présents inventeurs ont identifiées *in silico* dans des séquences codantes de la séquence du génome du palmier dattier (Al-Dous et al., Nature Biotechnology, 2011, 29: 521-527 - http://qatar-weill.cornell.edu/research/datepalmGenome/download.html). Ces 4 loci ont été sélectionnés parmi 47 pour leur capacité d'amplification et le polymorphisme qu'ils génèrent.

Les marqueurs PdCUC3-ssr1 et PdCUC3-ssr2 sont des séquences dinucléotidiques identifiées dans des gènes CUC (Adam et al., Molecular Biology and Evolution, 2010, 28: 1439-1454).

Les marqueurs PdAG1-ssr1 et PdAP3-ssrl sont des séquences dinucléotidiques identifiées par les présents inventeurs dans le gène AG1 et le gène AP3 (non publié) respectivement.

Le marqueur CpfM12 est un marqueur minisatellite du génome chloroplastique (Henderson et al., Conservation Genetics, 2006, 7: 213-223).

Dans les deux jeux de marqueurs mini- et micro-satellites spécifiques de la diversité génétique du palmier dattier, les marqueurs sont définis soit par leur motif et la séquence dans laquelle ils sont contenus, soit par leur motif et les séquences nucléotidiques conservées qui les flanquent en 5' et en 3'. Les termes "séquence nucléotidique", "séquence", et "séquence nucléique", sont ici employés indifféremment. Par ces termes, on entend désigner un enchaînement précis de nucléotides permettant de définir une région d'un acide nucléique, et pouvant correspondre aussi bien à un ADN double brin ou un ADN simple brin qu'à des produits de transcription de ces molécules d'ADN.

Les informations fournies ci-dessous pour définir chaque marqueur SSR d'un jeu de marqueurs décrit ici concernent le brin 5'→3' de la région du génome dans lequel se situe le SSR. Comme le reconnaîtra l'homme du métier, étant donné qu'une séquence SSR est une séquence d'ADN double brin, est englobé également ici le motif et le locus complémentaires (se trouvant sur le brin 3'→5').

Le marqueur mPdCIR010 est caractérisé en ce que dans le sens 5'→3', il a un motif (ga); est présent dans la séquence de numéro d'ordre GenBank AJ571673.1 (*Phoenix dactylifera* microsatellite DNA, clone CIR010) ; et est flanqué, en 5', par la séquence nucléotidique SEQ ID NO: 1, et en 3', par la séquence nucléotidique SEQ ID NO: 2.

Le marqueur mPdCIR015 est caractérisé en ce que dans le sens 5'→3', il a un motif (ga) ; est présent dans la séquence de numéro d'ordre GenBank AJ571674.1 (*Phoenix dactylifera* microsatellite DNA, clone CIR015) ; et est flanqué, en 5', par la séquence nucléotidique SEQ ID NO: 3, et en 3', par la séquence nucléotidique SEQ ID NO: 4.

Le marqueur mPdCIR016 est caractérisé en ce que dans le sens 5'→3', il a un motif (ga) ; est présent dans la séquence de numéro d'ordre GenBank AJ571675.1 (*Phoenix dactylifera* microsatellite DNA, clone CIR016) ; et est flanqué, en 5', par la séquence nucléotidique SEQ ID NO: 5, et en 3', par la séquence nucléotidique SEQ ID NO: 6.

Le marqueur mPdCIR025 est caractérisé en ce que dans le sens 5'→3', il a un motif (ga) ; est présent dans la séquence de numéro d'ordre GenBank AJ571676.1 (*Phoenix dactylifera* microsatellite DNA, clone CIR025) ; et est flanqué, en 5', par la séquence nucléotidique SEQ ID NO: 7, et en 3', par la séquence nucléotidique SEQ ID NO: 8.

Le marqueur mPdCIR032 est caractérisé en ce que dans le sens 5'→3', il a un motif (ga) ; est présent dans la séquence de numéro d'ordre GenBank AJ571677.1 (*Phoenix dactylifera* microsatellite DNA, clone CIR032) ; et est flanqué, en 5', par la séquence nucléotidique SEQ ID NO: 9, et en 3', par la séquence nucléotidique SEQ ID NO: 10.

Le marqueur mPdCIR035 est caractérisé en ce que dans le sens 5'→3', il a un motif (ga) ; est présent dans la séquence de numéro d'ordre GenBank AJ571678.1 (*Phoenix dactylifera* microsatellite DNA, clone CIR035) ; et est flanqué, en 5', par la séquence nucléotidique SEQ ID NO: 11, et en 3', par la séquence nucléotidique SEQ ID NO: 12.

Le marqueur mPdCIR057 est caractérisé en ce que dans le sens 5'→3', il a un motif (ga) ; est présent dans la séquence de numéro d'ordre GenBank AJ571682.1 (*Phoenix dactylifera* microsatellite DNA, clone CIR057) ; et est flanqué, en 5', par la séquence nucléotidique SEQ ID NO: 13, et en 3', par la séquence nucléotidique SEQ ID NO: 14.

Le marqueur mPdCIR063 est caractérisé en ce que dans le sens 5'→3', il a un motif (ga) ; est présent dans la séquence de numéro d'ordre GenBank AJ571683.1 (*Phoenix dactylifera* microsatellite DNA, clone CIR063) ; et est flanqué, en 5', par la séquence nucléotidique SEQ ID NO: 15, et en 3', par la séquence nucléotidique SEQ ID NO: 16.

Le marqueur mPdCIR078 est caractérisé en ce que dans le sens 5'→3', il a un motif (ga) ; est présent dans la séquence de numéro d'ordre GenBank AJ571685.1 (*Phoenix dactylifera* microsatellite DNA, clone CIR078) ; et est flanqué, en 5', par la séquence nucléotidique SEQ ID NO: 17, et en 3', par la séquence nucléotidique SEQ ID NO: 18.

Le marqueur mPdCIR085 est caractérisé en ce que dans le sens 5'→3', il a un motif (ga) ; est présent dans la séquence de numéro d'ordre GenBank AJ571686.1 (*Phoenix dactylifera* microsatellite DNA, clone CIR085) ; et est flanqué, en 5', par la séquence nucléotidique SEQ ID NO: 19, et en 3', par la séquence nucléotidique SEQ ID NO: 20.

Le marqueur CpfM12 est caractérisé en ce que dans le sens 5'→3', il a un motif (ctaactactata) ; est présent dans la séquence de numéro d'ordre GenBank EU043484.1 ; et est flanqué, en en 5', par la séquence nucléotidique SEQ ID NO: 21, et en 3', par la séquence nucléotidique SEQ ID NO: 22.

Le marqueur PdCUC3-ssr1 est caractérisé en ce que dans le sens 5'→3', il a un motif (gt+ga) ; est présent dans la séquence de numéro d'ordre GenBank HM622273.1 (*Phoenix dactylifera* cup-shaped cotyledon 3-related protein (CUC3) gene, partial cds) ; et est flanqué, en en 5', par la séquence nucléotidique SEQ ID NO: 23, et en 3', par la séquence nucléotidique SEQ ID NO: 24.

Le marqueur PdCUC3-ssr2 est caractérisé en ce que dans le sens 5'→3', il a un motif (ga) ; est présent dans la séquence de numéro d'ordre GenBank HM622273.1 (*Phoenix dactylifera* cup-shaped cotyledon 3-related protein (CUC3) gene, partial cds) ; et est flanqué, en en 5', par la séquence nucléotidique SEQ ID NO: 25, et en 3', par la séquence nucléotidique SEQ ID NO: 26.

Le marqueur PdAG1-ssr1 est caractérisé en ce que dans le sens 5'→3', il a un motif (ga) ; est présent dans la séquence de numéro d'ordre GenBank KC188338.1 ; et est flanqué, en en 5', par la séquence nucléotidique SEQ ID NO: 27, et en 3', par la séquence nucléotidique SEQ ID NO: 28.

Le marqueur PdAP3-ssr est caractérisé en ce que dans le sens 5'→3', il a un motif (ga) ; est présent dans la séquence de numéro d'ordre GenBank KC188337.1 ; et est flanqué, en en 5', par la séquence nucléotidique SEQ ID NO: 29, et en 3', par la séquence nucléotidique SEQ ID NO: 30.

Le marqueur mPdIRD13 est caractérisé en ce que dans le sens 5'→3', il a un motif (cac) ; est présent dans le scaffold PDK_20s1496731g002_1 (numéro d'ordre GenBank GL741615.1) ; et est flanqué, en en 5', par la séquence nucléotidique SEQ ID NO: 31, et en 3', par la séquence nucléotidique SEQ ID NO: 32 ;
Le marqueur mPdIRD31 est caractérisé en ce que dans le sens 5'→3', il a un motif (cca) ; est présent dans le scaffold PDK_20s1419261g003_1 (décrit dans http://qatar-weill.cornell.edu/research/datepalmGenome/download.html) et est flanqué, en en 5', par la séquence nucléotidique SEQ ID NO: 33, et en 3', par la séquence nucléotidique SEQ ID NO: 34.

Le marqueur mPdIRD33 est caractérisé en ce que dans le sens 5'→3', il a un motif (cag) ; est présent dans le scaffold PDK_20s1569281g001_1 (décrit dans http://qatar-weill.cornell.edu/research/datepalmGenome/download.html), et est flanqué, en en 5', par la séquence nucléotidique SEQ ID NO: 35, et en 3', par la séquence nucléotidique SEQ ID NO: 36.

Le marqueur mPdIRD40 est caractérisé en ce que dans le sens 5'→3', il a un motif (ccagtg) ; est présent dans le scaffold PDK_20s1327401g002_1 (décrit dans http://qatar-weill.cornell.edu/research/datepalmGenome/download.html); et est flanqué, en 5', par la séquence nucléotidique SEQ ID NO: 37, et en 3', par la séquence nucléotidique SEQ ID NO: 38.

### II - Utilisation des Jeux de Marqueurs pour identifier les Génotypes du Palmier Dattier et/ou les Cultivars du Palmier Dattier

Comme l'ont démontré les présents inventeurs, les jeux de marqueurs moléculaires décrits ci-dessus peuvent être utilisés pour identifier les génotypes de palmiers dattiers. Généralement, une méthode d'identification décrite ici se fait par détection, à l'aide d'un jeu de marqueurs, d'un polymorphisme SSR dans le génome d'un individu de palmier dattier, et comprend, pour chacun des marqueurs mini- ou micro-satellites du jeu, l'amplification d'une région du génome du palmier dattier à tester, la région comprenant la séquence SSR.

### Echantillons de Palmier Dattier

Dans une méthode décrite ici, l'étape consistant à amplifier une région (ou portion) du génome du palmier dattier comprenant un motif SSR répété est réalisée sur un échantillon du palmier dattier à tester, et préférablement sur de l'ADN génomique extrait de l'échantillon du palmier dattier. On entend ici par "ADN génomique", l'ADN du génome nucléaire (c'est-à-dire l'ADN génomique contenu dans le noyau) et du génome chloroplastique (c'est-à-dire l'ADN génomique contenu dans les chloroplastes).

Tout échantillon de dattier contenant de l'ADN génomique peut donc être utilisé dans la mise en oeuvre de la méthode décrite. Par exemple, l'ADN génomique peut être extrait de protoplastes, de cals, d'embryons, de feuilles, de stipes, de racines, de rejets ou de boutures de dattiers.

Les méthodes d'extraction d'ADN de tissus biologiques sont bien connues dans l'art (voir par exemple, Sambrook et al., "Molecular Cloning - A Laboratory Manual", Cold Spring Harbor Laboratory Press, 1989). Il existe également de nombreux kits disponibles dans le commerce (par exemple chez BD Biosciences Clontech (Palo Alto, CA), Epicentre Technologies (Madison, WI), Gentra Systems, Inc. (Minneapolis, MN), MicroProbe Corp. (Bothell, WA), Organon Teknika (Durham, NC), et Qiagen Inc. (Valencia, CA)) qui peuvent être utilisés pour extraire l'ADN d'échantillons de dattier.

### Amplification de la Séquence Microsatellite

Comme le reconnaîtra l'homme du métier, dans la mise en oeuvre d'une méthode décrite ici, l'amplification d'une portion du génome du palmier dattier comprenant une séquence microsatellite donnée peut être effectuée par n'importe quelle technique appropriée connue dans l'art, la technique utilisée n'étant pas un facteur limitant.

Préférablement, dans une méthode décrite ici, les réactions d'amplification d'ADN génomique sont conduites par amplification PCR (« Polymerase Chain Reaction » ou « Amplification en Chaîne par Polymérase » en français) (Mullis and Faloona, Methods Enzymol., 1987, 155: 355-350) qui offre l'avantage d'analyser les marqueurs moléculaires en un temps court tout en utilisant des concentrations faibles d'ADN. Les régions flanquantes des microsatellites servent d'amorces lors de la PCR. Etant donné que ces régions sont conservées, une paire d'amorces spécifiques de ces régions flanquantes amplifie spécifiquement ce seul micro satellite (et pas un autre).

Les différents amplicons (ou produits d'amplification) générés à partir d'une région microsatellite donnée ont des tailles caractéristiques et reproductibles. La variation des tailles des produits PCR est causée par des différences dans le nombre de répétition du motif du microsatellite. Ces tailles sont donc indicatives de la (si l'échantillon de palmier dattier est homozygote) ou des (si l'échantillon de palmier dattier est hétérozygote) longueurs de la séquence microsatellite chez l'individu.

L'homme du métier sait comment sélectionner les conditions optimales (température, durée et nombre de cycles, pH, et concentrations des réactifs) pour effectuer une amplification par PCR ("PCR Protocols: A Guide to Methods and Applications", M.A. Innis (Ed.), 1990, Academic Press: New York; "PCR Strategies", M.A. Innis (Ed.), 1995, Academic Press: New York; "Polymerase chain reaction: basic principles and automation in PCR: A Practical Approach", McPherson et al. (Eds.), 1991, IRL Press: Oxford; R.K. Saiki et al., Nature, 1986, 324: 163-166).

### Amorces et Sondes pour l'Amplification d'une Portion du Génome du Palmier Dattier Comprenant une Séquence SSR

A partir de la séquence nucléotidique d'un scaffold ou autre séquence génomique où se situe un microsatellite d'un jeu de marqueurs moléculaires décrits ici et de la position de ce microsatellite dans ce scaffold ou cette séquence, l'homme du métier sait concevoir des amorces SSR appropriées à l'amplification d'une portion du génome du palmier dattier qui comprend la séquence microsatellite.

Les termes "amorce" et "amorce PCR" sont ici utilisés de façon interchangeable et désignent un oligonucléotide qui est capable d'agir comme point de départ à la synthèse d'un produit d'amplification, lorsqu'il est placé dans des conditions appropriées d'amplification (par exemple, concentration en sel, température et pH) en présence de nucléotides et d'un agent de polymérisation d'acide nucléique (par exemple une ADN polymérase). Une amorce décrite ici comprend un oligonucléotide contenant avantageusement entre 5 et 50 nucléotides, généralement entre 15 et 50 nucléotides, préférablement entre 20 et 35 nucléotides, plus préférablement encore entre 20 et 25 nucléotides (par exemple 20, 21, 22, 23, 24 ou 25 nucléotides).

Le terme "amorce SSR" fait référence à une amorce qui est spécifique d'une région flanquante ou adjacente de la région microsatellite et qui, en combinaison avec une autre amorce SSR, est capable d'amplifier spécifiquement la région microsatellite. L'amplification est généralement réalisée à l'aide d'une paire d'amorces SSR comprenant une amorce forward (ou « sens ») et une amorce reverse (ou « antisens ») qui chacune s'hybride sur un des deux brins de l'ADN génomique.

Préférablement, une amorce SSR forward décrite ici comprend, ou est constituée par, une séquence de 15 à 50 nucléotides consécutifs, préférablement de 20 à 35 nucléotides consécutifs, plus préférablement encore de 20 à 25 nucléotides consécutifs, de la séquence d'au plus 1000 nucléotides flanquant, en 5', la région microsatellite décrite ici. Préférablement, une amorce SSR reverse décrite ici comprend, ou est constituée par, une séquence de 15 à 50 nucléotides consécutifs, préférablement de 20 à 35 nucléotides consécutifs, plus préférablement encore de 20 à 25 nucléotides consécutifs, de la séquence complémentaire de la séquence d'au plus 1000 nucléotides flanquant, en 3', la région microsatellite décrite ici.

Par "séquence complémentaire" d'une séquence nucléotidique donnée, on entend une séquence qui forme, par hybridation, un duplex stable avec ladite séquence nucléotidique. Le terme "séquence complémentaire" désigne aussi bien la séquence complémentaire présentée dans le sens 3'→5' que la séquence complémentaire présentée dans le sens 5'→3' (c'est-à-dire la séquence complémentaire inverse). Par "hybridation" on entend ici l'association tête bêche de deux polynucléotides simple brin par des appariements Watson-Crick (A-T, G-C). Dans certains cas, l'hybridation est parfaite, c'est-à-dire que les séquences sont totalement complémentaires. Ainsi, par exemple, le terme "la séquence complémentaire de la séquence SEQ ID NO: 1" est la séquence nucléotidique qui est parfaitement ou totalement complémentaire de SEQ ID NO: 1.

Ainsi, dans la mise en oeuvre d'une méthode décrite ici, l'amplification d'une région du génome du palmier dattier comprenant la séquence du microsatellite de motif (ga) du marqueur mPdCIR010 est préférablement réalisée à l'aide d'une paire d'amorces SSR comprenant :
- une amorce SSR forward de séquence SEQ ID NO: 1 (ACCCCGGACGTGAGGTG), et
- une amorce SSR reverse de séquence SEQ ID NO: 2 (CGTCGATCTCCTCCT TTGTCTC).

L'amplification d'une région du génome du palmier dattier comprenant la séquence du microsatellite de motif (ga) du marqueur mPdCIR015 est préférablement réalisée à l'aide d'une paire d'amorces SSR comprenant :
- une amorce SSR forward de séquence SEQ ID NO: 3 (AGCTGGCTCCTCCCTTCTTA), et
- une amorce SSR reverse de séquence SEQ ID NO: 4 (GCTCGGTTGGACTTGTTCT).

L'amplification d'une région du génome du palmier dattier comprenant la séquence du microsatellite de motif (ga) du marqueur mPdCIR016 est préférablement réalisée à l'aide d'une paire d'amorces SSR comprenant :
- une amorce SSR forward de séquence SEQ ID NO: 5 (AGCGGGAAATGAAAAGGTAT), et
- une amorce SSR reverse de séquence SEQ ID NO: 6 (ATGAAAACGTGCCAAATGTC).

L'amplification d'une région du génome du palmier dattier comprenant la séquence du microsatellite de motif (ga) du marqueur mPdCIR025 est préférablement réalisée à l'aide d'une paire d'amorces SSR comprenant :
- une amorce SSR forward de séquence SEQ ID NO: 7 (GCACGAGAAGGCTTATAGT), et
- une amorce SSR reverse de séquence SEQ ID NO: 8 (CCCCTCATTAGGATTCTAC).

L'amplification d'une région du génome du palmier dattier comprenant la séquence du microsatellite de motif (ga) du marqueur mPdCIR032 est préférablement réalisée à l'aide d'une paire d'amorces SSR comprenant :
- une amorce SSR forward de séquence SEQ ID NO: 9 (CAAATCTTTGCCGTGAG), et
- une amorce SSR reverse de séquence SEQ ID NO: 10 (GGTGTGGAGTAATCATGTAGTAG).

L'amplification d'une région du génome du palmier dattier comprenant la séquence du microsatellite de motif (ga) du marqueur mPdCIR035 est préférablement réalisée à l'aide d'une paire d'amorces SSR comprenant :
- une amorce SSR forward de séquence SEQ ID NO: 11 (ACAAACGGCGATGGGATTAC), et
- une amorce SSR reverse de séquence SEQ ID NO: 12 (CCGCAGCTCACCTCTTCTAT).

L'amplification d'une région du génome du palmier dattier comprenant la séquence du microsatellite de motif (ga) du marqueur mPdCIR057 est préférablement réalisée à l'aide d'une paire d'amorces SSR comprenant :
- une amorce SSR forward de séquence SEQ ID NO: 13 (AAGCAGCAGCCCTTCCGTAG), et
- une amorce SSR reverse de séquence SEQ ID NO: 14 (GTTCTCACTCGCCCAAAAATAC).

L'amplification d'une région du génome du palmier dattier comprenant la séquence du microsatellite de motif (ga) du marqueur mPdCIR063 est préférablement réalisée à l'aide d'une paire d'amorces SSR comprenant :
- une amorce SSR forward de séquence SEQ ID NO: 15 (CTTTTATGTGGTCTGAGAGA), et
- une amorce SSR reverse de séquence SEQ ID NO: 16 (TCTCTGATCTTGGGTTCTGT).

L'amplification d'une région du génome du palmier dattier comprenant la séquence du microsatellite de motif (ga) du marqueur mPdCIR078 est préférablement réalisée à l'aide d'une paire d'amorces SSR comprenant :
- une amorce SSR forward de séquence SEQ ID NO: 17 (TGGATTTCCATTGTGAG), et
- une amorce SSR reverse de séquence SEQ ID NO: 18 (CCCGAAGAGACGCTATT).

L'amplification d'une région du génome du palmier dattier comprenant la séquence du microsatellite de motif (ga) du marqueur mPdCIR085 est préférablement réalisée à l'aide d'une paire d'amorces SSR comprenant :
- une amorce SSR forward de séquence SEQ ID NO: 19 (GAGAGAGGGTGGTGTTATT), et
- une amorce SSR reverse de séquence SEQ ID NO: 20 (TTCATCCAGAACCACAGTA).

L'amplification d'une région du génome du palmier dattier comprenant la séquence du minisatellite de motif (ctaactactata) du marqueur CpfM12 est préférablement réalisée à l'aide d'une paire d'amorces SSR comprenant :
- une amorce SSR forward de séquence SEQ ID NO: 21 (CCGCCCACGATGAAGTAATGTA), et
- une amorce SSR reverse de séquence SEQ ID NO: 22 (GTCACGGGTTCAAATCCTGTCTC).

L'amplification d'une région du génome du palmier dattier comprenant la séquence du microsatellite de motif (gt+ga) du marqueur PdCUC3-ssr1 est préférablement réalisée à l'aide d'une paire d'amorces SSR comprenant :
- une amorce SSR forward de séquence SEQ ID NO: 23 (CGTGGACTCATGACTCGCATGTCC), et
- une amorce SSR reverse de séquence SEQ ID NO: 24 (GGTCCTTGCCGGTGGCCTTC).

L'amplification d'une région du génome du palmier dattier comprenant la séquence du microsatellite de motif (ga) du marqueur PdCUC3-ssr2 est préférablement réalisée à l'aide d'une paire d'amorces SSR comprenant :
- une amorce SSR forward de séquence SEQ ID NO: 25 (ACATTGCTCTTTTGCCATGGGCT), et
- une amorce SSR reverse de séquence SEQ ID NO: 26 (CGAGCAGGTGGGGTTCGGGT).

L'amplification d'une région du génome du palmier dattier comprenant la séquence du microsatellite de motif (ga) du marqueur PdAG1-ssr1 est préférablement réalisée à l'aide d'une paire d'amorces SSR comprenant :
- une amorce SSR forward de séquence SEQ ID NO: 27 (TCTGATTTCGTTTACTTCTTAGGA), et
- une amorce SSR reverse de séquence SEQ ID NO: 28 (TTCATATTCAGTTGTCGGGTGTA).

L'amplification d'une région du génome du palmier dattier comprenant la séquence du microsatellite de motif (ga) du marqueur PdAP3-ssr est préférablement réalisée à l'aide d'une paire d'amorces SSR comprenant :
- une amorce SSR forward de séquence SEQ ID NO: 29 (GAGAAATAGAGAGCTGTGCAAG), et
- une amorce SSR reverse de séquence SEQ ID NO: 30 (CTGCAGTACTCGGAGAACTTG).

L'amplification d'une région du génome du palmier dattier comprenant la séquence du microsatellite de motif (cac) du marqueur mPdIRD13 est préférablement réalisée à l'aide d'une paire d'amorces SSR comprenant :
- une amorce SSR forward de séquence SEQ ID NO: 31 (GCGGAGACAGGAGATGGTAA), et
- une amorce SSR reverse de séquence SEQ ID NO: 32 (CTTGACTGCTTCTGCTGCTG).

L'amplification d'une région du génome du palmier dattier comprenant la séquence du microsatellite de motif (cca) du marqueur mPdIRD31 est préférablement réalisée à l'aide d'une paire d'amorces SSR comprenant :
- une amorce SSR forward comprenant, ou constituée de, entre 15 et 50 nucléotides consécutifs de la séquence d'au plus 1000 nucléotides flanquant, en 5', la position 29073 dans le scaffold PDK_20s1419261g003_1 (http://qatar-weill.cornell.edu/research/datepalmGenome/download.html), et
- une amorce SSR reverse comprenant, ou constituée de, entre 15 et 50 nucléotides consécutifs de la séquence complémentaire de la séquence d'au plus 1000 nucléotides flanquant, en 3', la position 29093 dans le scaffold PDK_20s1419261g003_1.

La séquence d'au plus 1000 nucléotides peut comporter n'importe quel nombre de nucléotides préférablement compris entre 1000 et 250, par exemple 1000, 900, 800, 700, 600, 500, 400, 300, ou 250.

Par exemple, une paire d'amorces pour l'amplification d'une région du génome du palmier dattier comprenant la séquence du microsatellite de motif (cca) du marqueur mPdIRD31 peut comprendre une amorce SSR forward de séquence SEQ ID NO: 33 (GCAGGTGGACTGCAAAATCT) et une amorce SSR reverse de séquence SEQ ID NO: 34 (CTATTGGGGTGCTGATCCAT).

L'amplification d'une région du génome du palmier dattier comprenant la séquence du microsatellite de motif (cag) du marqueur mPdIRD33 est préférablement réalisée à l'aide d'une paire d'amorces SSR comprenant :
- une amorce SSR forward comprenant, ou constituée de, entre 15 et 50 nucléotides consécutifs de la séquence d'au plus 1000 nucléotides flanquant, en 5', la position 5206 dans le scaffold PDK_20s1569281g001_1 (http://qatar-weill.cornell.edu/research/datepalmGenome/download.html), et
- une amorce SSR reverse comprenant, ou constituée de, entre 15 et 50 nucléotides consécutifs de la séquence complémentaire de la séquence d'au plus 1000 nucléotides flanquant, en 3', la position 5226 dans le scaffold PDK_20s1569281g001_1.

La séquence d'au plus 1000 nucléotides peut comporter n'importe quel nombre de nucléotides préférablement compris entre 1000 et 250, par exemple 1000, 900, 800, 700, 600, 500, 400, 300, ou 250.

Par exemple, une paire d'amorces pour l'amplification d'une région du génome du palmier dattier comprenant la séquence du microsatellite de motif (cag) du marqueur mPdIRD33 peut comprendre une amorce SSR forward de séquence SEQ ID NO: 35 (GGAGCATACAGTGGGTTTGC) et une amorce SSR reverse de séquence SEQ ID NO: 36 (CAGCCTGGGAATGAGGATAG).

L'amplification d'une région du génome du palmier dattier comprenant la séquence du microsatellite de motif (ccagtg) du marqueur mPdIRD40 est préférablement réalisée à l'aide d'une paire d'amorces SSR comprenant :
- une amorce SSR forward comprenant, ou constituée de, entre 15 et 50 nucléotides consécutifs de la séquence d'au plus 1000 nucléotides flanquant, en 5', la position 16193 dans le scaffold PDK_20s1327401g002_1 (http://qatar-weill.cornell.edu/research/datepalmGenome/download.html), et
- une amorce SSR reverse comprenant, ou constituée de, entre 15 et 50 nucléotides consécutifs de la séquence complémentaire de la séquence d'au plus 1000 nucléotides flanquant, en 3', la position 16216 dans le scaffold PDK_20s1327401g002_1.

La séquence d'au plus 1000 nucléotides peut comporter n'importe quel nombre de nucléotides préférablement compris entre 1000 et 250, par exemple 1000, 900, 800, 700, 600, 500, 400, 300, ou 250.

Par exemple, une paire d'amorces pour l'amplification d'une région du génome du palmier dattier comprenant la séquence du microsatellite de motif (ccagtg) du marqueur mPdIRD40 peut comprendre une amorce SSR forward de séquence SEQ ID NO: 37 (GAGAGATGCGTCAGGGAATC), et une amorce SSR reverse de séquence SEQ ID NO: 38 (CCAGAATCTTCCAAGCAAGC).

Une amorce décrite ici est marquée de manière à permettre sa détection (et, par conséquent, celle des produits d'amplification ou amplicons obtenus par PCR). Différents types de marquage, connus de l'homme du métier, peuvent être utilisés (marquage radioactif, fluorescence, chimiluminescence, marquage de type M13, etc...). Le marqueur peut être intégré dans l'oligonucléotide composant l'amorce, ou associé à cet oligonucléotide (par exemple par liaison covalente). Par "amorce marquée" ou "sonde", on entend donc désigner une amorce qui contient, ou qui est associée ou liée (par exemple de façon covalente) à un marqueur détectable.

Les amorces peuvent être préparées par n'importe quelle méthode appropriée connue de l'homme du métier, choisie en particulier parmi les méthodes classiques de synthèse d'oligonucléotides comme les méthodes de synthèse sur phase solide. Les amorces décrites ici peuvent par exemple être préparées à l'aide d'un synthétiseur d'oligonucléotides (comme ceux commercialisés, par exemple, par Applied Biosystems ou GE Healthcare). De même, des méthodes pour marquer les oligonucléotides sont connues dans l'art.

En plus des amorces décrites ici (ou tout autre amorce qui peut se déduire des informations fournies), on considère aussi ici leur utilisation pour identifier le génotype (et/ou cultivar) du palmier dattier par amplification d'une portion du génome du palmier dattier qui comprend une séquence microsatellite décrite plus haut.

### Analyse des Amplicons

Dans une méthode d'identification du génotype du palmier dattier utilisant un des deux jeux de marqueurs moléculaires décrits ici, après amplification, pour chaque marqueur du jeu, d'une portion du génome du palmier dattier comprenant la séquence microsatellite, les amplicons sont analysés pour déterminer le génotype du palmier dattier testé.

L'analyse des amplicons peut se faire par n'importe quelle méthode. L'analyse comprend généralement la séparation des amplicons en fonction de leur taille (et donc de la taille de la séquence microsatellite). La séparation peut, par exemple, être effectuée par électrophorèse (une technique qui permet de séparer les fragments d'ADN en fonction de leur charge électrique et de leur taille) sur gel d'agarose ou sur gel de polyacrylamide. La séparation est suivie de la détection des fragments séparés par coloration au bromure d'éthidium ou à l'argent, ou encore par détection du marqueur détectable de l'amorce (par fluorescence, radioactivité, etc...).

Pour déterminer le génotype du palmier dattier testé, les tailles des amplicons obtenus peuvent être comparées aux tailles des amplicons obtenus par amplification, effectuée dans les mêmes conditions, de l'ADN génomique d'un palmier dattier contrôle. Par "palmier dattier contrôle", on désigne ici un palmier dattier dont le génotype (origine, variété, cultivar, etc.) est connu.

L'étape de comparaison des tailles des amplicons obtenus à partir de l'échantillon de palmier dattier testé peut comprendre l'utilisation de plusieurs palmiers dattiers contrôles. En particulier, l'étape de comparaison des tailles des amplicons obtenus, pour le jeu de 19 ou le jeu de 7 marqueurs, à partir de l'échantillon de palmier dattier peut comprendre la comparaison avec les tailles des amplicons obtenus, pour le même jeu de marqueurs, à partir des génotypes utilisés par les inventeurs, et rassemblés dans la base de données PhoenixDB http://phoenixdb.mpl.ird.fr (login : phoenix ; mot de passe : s7RSFV).

Pour déterminer le génotype du palmier dattier testé, les amplicons peuvent, après séparation en fonction de leur taille, être séquencées pour déterminer le ou les nombres de répétitions du motif SSR dans le génome du palmier dattier testé. Les méthodes de séquençage de fragments d'ADN sont connues dans l'art. En particulier, il est possible d'utiliser des séquenceurs automatiques comme ceux disponibles chez Beckman, Applied BioSystems, or LiCor Biosciences.

### Identification du Génotype et/ou du Cultivar du Palmier Dattier Testé

Comme indiqué plus haut, le jeu de 19 marqueurs permet d'analyser 100% des génotypes de palmiers dattiers. Les palmiers dattiers testés par une méthode décrite ici peuvent être originaires de n'importe quelle région du monde, en particulier d'Israël, de Libye, de Tunisie, de Mauritanie, d'Algérie, du Pakistan, d'Irak, du Soudan, du Cap Vert, d'Espagne, du Niger, du Maroc, d'Italie, d'Oman, de Djibouti, d'Iran, d'inde, de Thaïlande, ou du Vietnam.

En particulier, le jeu de 19 marqueurs permet d'identifier les génotypes et variétés de palmiers dattiers de l'espèce *Phoenix dactylifera L.* choisis parmi Aqdool, Taj, Lemsi mâle, Edaghed, Halwa, Halwaya, Gadri (Yellow Seed), Ashrasi, Badrayah, Gadri, Kentichi, Mech Degla, Benosh, Besser Helou, Khmag, Laloo, Awreeq, Dguel Mghas, Amreer, Dokhar, Aréchti, Deglet Jdir, Barakawi, Elche, Outaghsaït, Buféa, Adam Goas, Deglet Wlad Mahmoud, Ghars tunisien, Gadri, Jonah, Akerbouche, Baydir, Tinjdel, Abbad, Ali puri chohara, Timakur, Bou Feggous marocain, Bou Feggous algérien (Fagous), Thorry, Alig tunisien, Peggy Ann (proche Thorry), Kenta algérien, La Confitera, Medjoul, Back Cross Medjool 4ème G, Romana I, Bidh-Hmam, Kenta tunisien, Chikh, Chikh (Hamuri), Chikh (Mhammed), Hamraya III, Alig algérien, Alig (Bu'Rus), Alig (Ftimi), Tamazouart, Deglet Gurara, Lagou, Bayd Hmam, Bitamoda, Bent Cherk, Bent Cherk (Cherka), Bukhannus, Tinasser, Degla Baida, Gondaila, Cheddakh, Deglet Hassan, Timedjwel, Takhoudrayt, Bou Feggous tunisien, Bessir, Bouskri, Deglet Bey, Deglet Bey (Menakher), Sekani, Talees, Dogna, Tinhamour, Taghiyat, Hmara tunisien, Gasbi, Me'udiya, Tadmamt, Tindukan, Aman, Takerboucht, Tazizawt, Ouarglia, Aghares, Goundi, Peli Sundar, Khara Basra, Amir Hajj, Mobai (Deri wala), Mobai (Dost Muhammadi Wala), Mobai, Horra, Rachna, Fasli, Shakri, Zaidi (Danda), Zaidi (Halimi), Zaidi (Masri), Zaidi (not Karblain), Zaidi, Zaidi, Dhakki, Dhakki (Congon), Khalas, Seib, Naghal (Gajjar/Gujjar), Naghal, Lolo, Bahlani, Gabeeni Mobai, Romana II, Dokhar, Adam Alkag, Aseel (Angor), Aseel, Aseel (Dhakki Zard), Aseel (Khurma), Chohara, Qasab, Badmi, Barhi, Hardasht, Hardasht (Jwana), Jeremiah, Khastawi, Tadala, Medina Kaylanya, Braim, Makran, Mcharret, Taghera, Saidi, Tati, Timrisa, Deglet Nour, Deglet Nour mère des Pa, Adam Hror, Eve, D'quel El Hadj, Banekhluf, Bukezzine, Ahmar, Rukkan, Shado, Gulistan, Khurmo, Halawi (Champa Kali), Halawi (Gulistan), Halawi, Halawi (Hillawi), Halawi (Neelum), Halawi (Pela Dora), Halawi (Zohdi), Khupra, Adam Timu, Choti Sandori, Chataya, Tawadan, Yellow Lulu, Bouldjib, Aghaliane, Ain Zbib, Tazerzist, Deglet Jito, Adam Bchir, Ma'tug, Ajwa, Chapshoq, Khalass Oman, Zerin, Surkh Begum Jungi, Gurbago, Karblain, Karblain (Ko-harba), Sajho Wali, Kozan Abad, Basra, Tabarzal, Pashpag, Pasht Kharoch, Black Bou Sthammi, Shamran, Otakanr, Kesba, Kesba (Sokrya), Zardo, Deglet Mech'a, Begum Jungi II, Kokna, Zard Begum Jungi, Dhaidi wali, Bounaringa, Hawwa wali, Khalass, Akhrot, Fard (Dhandari), Fard (Farz), Fard, Ajoujil, Qantar, Jaman, Ko-harba, Goknah, Azizaou (nom arabe : Adam Zrak), Azizaou (nom arabe : Amazigh), Tafeziwin, Adam Deglet Noir, Timliha, Adam Esof, Dguel Sidi Khlil, Adam Araw, Gadri (Red), Ain Zbib, Ououchtt, Tinhud, Tawragha, Tissibi, Ighes N'wagada, Tilemsu, Kasho wali, Desi, Halini, Hilali, Hussaini, Hussaini (Jan-sohar), Zard Kharoch, Bahram, Naz Tabakki, Saylani, Lasora, Khadrawy, Bdmalki, Bdmalki (Sarmadti), Maktoom, Kharoch, Jhajhri, Roghni, Gharas, Surkh, Noor Muhammadi Much, Mathusaleh, Aghammu, Mozati, Surkh Dhakki, Bent Qbala, Nasser Oussaleh, Bawa'adhim, Timliha, Tifazwin, Tanslit, Hmira, Taghera, Tgaza, Asemmat, Adam Bulla, Hamraya I, Be-Rehmi, Tinrigh, Khadri, Tazarzayet, Ebrea, Savi Khaji, Andekly, Begum Jungi, Litima, Harthan (Aharthan), Harthan (Oumazer), Klair, Timjouhart, red Lulu, Ouzamig, Tachlilat, Dimolo, Tawraghet, Tazougart, Tinicine, Hamraya II, Angou (= Algerian Ghars), Ghars, Tanteboucht, Tanteboucht (El Kayed), Abdel Azzaz, Bacheir, Tantabecht, Zoq El Moggar, Taramount, Bahdid, El Gachouche, D'quel M'rass, Takermoust, Tizouyadj, Zaghraya, Ratti Khajji, Begun, et Nisri, Dfor Lgot.

Ce jeu de marqueurs permet également d'identifier les génotypes de palmiers de l'espèce *Phoenix canariensi, Phoenix atlantica, Phoenix acaulis, Phoenix loureiroi, Phoenix andamanensis, Phoenix pusilla, Phoenix reclinata, Phoenix caespitosa, Phoenix roebelenii, Phoenix paludosa, Phoenix sylvestris,* et *Phoenix theophrasti.*

Le jeu de 7 marqueurs décrit ici permet d'analyser 99% des cultivars de palmier dattier. En particulier, le jeu de 7 marqueurs permet d'identifier les cultivars de *Phoenix dactylifera L.* choisis parmi les cultivars originaires de l'Algérie tels que les cultivars Adam Hror, Aghares, Ajoujil, Akerbouche, Bent Qbala, Chikh, Degla Baida, Deglet Jdir, Dfor Lgot, Dguel Mghas, Takerboucht, Takermoust, Tanslit, Tanteboucht; Tati, Tgaza, Thorry, Timjouhart, Tinasser, Tissibi, et Zaghraya ; les cultivars originaires de l'Arabie tels que les cultivars Ajwa et Khalass ; les cultivars originaires de l'Espagne tels que le cultivar La Confitera ; les cultivars originaires de l'Irak tels que les cultivars Ashrasi, Badmi, Bahram, Barhi, Basra, Bdmalki, Benosh, Halawi, Khadrawi, Khara Basra, Khastawi, Maktoom, Saylani, et Zaidi ; les cultivars originaires de l'Italie tels que les cultivars Ebrea et Romana ; les cultivars originaires de la Lybie tels que les cultivars Amreer, Aqdool, Awreeq, Khmag, Taghiyat, et Talees ; les cultivars originaires du Maroc tels que les cultivars Black Bou Sthammi, Bou Feggous marocain, Bouskri et Medjoul ; les cultivars originaires de Mauritanie tels que les cultivars Ahmar, Edaghed et Sekani ; les cultivars originaires d'Oman tels que les cultivars Bounaringa, Fard, Hilali et Naghal ; les cultivars originaires du Pakistan tels que les cultivars Ali puri chohara, Aseel, Begun, Dhakki, Halini, Hardasht, Hussaini, Karblain, Kozan Abad, Mobai, Mozati, Nisri, Pashpag, Rukkan, Sajho Wali, Shakri, Shamran, et Wahn wali ; les cultivars originaires du Soudan tels que les cultivars Barakawi, Bitamoda, Dogna et Gondaila ; les cultivars originaires de Tunisie tels que les cultivars Alig algérien, Alig tunisien, Aman, Angou (= Algerian Ghars), Aréchti, Besser Helou, Bessir, Bidh-Hmam, Bou Feggous tunisien, Cheddakh, Deglet Bey, Deglet Nour, Gasbi, Ghars tunisien, Goundi, Hmara tunisien, Horra, Kenta tunisien, Kentichi, Lagou, et Tazerzist.

### III - Kits d'Identification du Génotype du Palmier Dattier

Sont également décrits ici des kits comprenant du matériel utile pour la mise en oeuvre d'une méthode exposée ici. En particulier sont décrits ici des kits pour l'identification du génotype du palmier dattier et/ou l'identification du cultivar du palmier dattier, contenant du matériel permettant de détecter, dans le génome du palmier dattier, le polymorphisme de 19 séquences microsatellites ou de 7 séquences microsatellites.

En général, un kit comprend au moins un jeu de paires d'amorces SSR décrites ici permettant l'amplification de chacun des (19 ou 7) marqueurs d'un jeu décrit ici. Un kit peut être conçu pour être utilisé avec une technique particulière d'amplification, en particulier une technique de PCR.

En particulier, le kit peut comprendre les paires d'amorces suivantes :
- une amorce SSR forward de séquence SEQ ID NO: 1 et une amorce SSR reverse de séquence SEQ ID NO: 2 ;
- une amorce SSR forward de séquence SEQ ID NO: 3 et une amorce SSR reverse de séquence SEQ ID NO: 4 ;
- une amorce SSR forward de séquence SEQ ID NO: 5 et une amorce SSR reverse de séquence SEQ ID NO: 6 ;
- une amorce SSR forward de séquence SEQ ID NO: 7 et une amorce SSR reverse de séquence SEQ ID NO: 8 ;
- une amorce SSR forward de séquence SEQ ID NO: 9 et une amorce SSR reverse de séquence SEQ ID NO: 10;
- une amorce SSR forward de séquence SEQ ID NO: 11 et une amorce SSR reverse de séquence SEQ ID NO: 12 ;
- une amorce SSR forward de séquence SEQ ID NO: 13 et une amorce SSR reverse de séquence SEQ ID NO: 14 ;
- une amorce SSR forward de séquence SEQ ID NO: 15 et une amorce SSR reverse de séquence SEQ ID NO: 16 ;
- une amorce SSR forward de séquence SEQ ID NO: 17 et une amorce SSR reverse de séquence SEQ ID NO: 18 ;
- une amorce SSR forward de séquence SEQ ID NO: 19 et une amorce SSR reverse de séquence SEQ ID NO: 20 ;
- une amorce SSR forward de séquence SEQ ID NO: 21 et une amorce reverse de séquence SEQ ID NO: 22 ;
- une amorce SSR forward de séquence SEQ ID NO: 23 et une amorce SSR reverse de séquence SEQ ID NO: 24 ;
- une amorce SSR forward de séquence SEQ ID NO: 25 et une amorce SSR reverse de séquence SEQ ID NO: 26 ;
- une amorce forward de séquence SEQ ID NO: 27 et une amorce SSR reverse de séquence SEQ ID NO: 28 ;
- une amorce SSR forward de séquence SEQ ID NO: 29 et une amorce SSR reverse de séquence SEQ ID NO: 30 ;
- une amorce SSR forward de séquence SEQ ID NO: 31 et une amorce SSR reverse de séquence SEQ ID NO: 32 ;
- une amorce SSR forward de séquence SEQ ID NO: 33 et une amorce SSR reverse de séquence SEQ ID NO: 34 ;
- une amorce SSR forward de séquence SEQ ID NO: 35 et une amorce SSR reverse de séquence SEQ ID NO: 36 ; et
- une amorce SSR forward de séquence SEQ ID NO: 37 et une amorce SSR reverse de séquence SEQ ID NO: 38.

Alternativement, le kit peut comprendre les paires d'amorces suivantes :
- une amorce SSR forward de séquence SEQ ID NO: 17 et une amorce SSR reverse de séquence SEQ ID NO:18 ;
- une amorce SSR forward de séquence SEQ ID NO: 21 et une amorce SSR reverse de séquence SEQ ID NO:22 ;
- une amorce SSR forward de séquence SEQ ID NO: 25 et une amorce SSR reverse de séquence SEQ ID NO: 26 ;
- une amorce SSR forward de séquence SEQ ID NO: 29 et une amorce SSR reverse de séquence SEQ ID NO: 30 ;
- une amorce SSR forward de séquence SEQ ID NO: 33 et une amorce SSR reverse de séquence SEQ ID NO: 34 ;
- une amorce SSR forward de séquence SEQ ID NO: 35 et une amorce SSR reverse de séquence SEQ ID NO: 36 ; et
- une amorce SSR forward de séquence SEQ ID NO: 37 et une amorce SSR reverse de séquence SEQ ID NO: 38.

Il est entendu qu'un kit peut comprendre les deux jeux de paires d'amorces.

Un kit peut en outre comprendre des réactifs ou solutions d'extraction de l'ADN génomique de l'échantillon de palmier dattier, des réactifs ou solutions d'amplification PCR, des réactifs ou solutions de séparation d'amplicons en fonction de leur taille, des réactifs ou solutions de séquençage, et/ou des moyens de détection. Des protocoles pour utiliser ces réactifs et/ou solutions peuvent être inclus dans le kit.

Les différents composants du kit peuvent être fournis sous forme solide (par exemple sous forme lyophilisée) ou sous forme liquide. Un kit peut éventuellement comprendre un récipient contenant chacun des réactifs ou solutions, et/ou des récipients pour réaliser certaines étapes de la méthode décrite ici.

Un kit pourra également comprendre des instructions pour mettre en oeuvre la méthode décrite ici pour détecter, dans le génome de palmiers dattiers, le polymorphisme SSR de chacun des 19 ou des 7 marqueurs d'un jeu. Les instructions pour effectuer une méthode décrite ici peuvent comprendre des instructions pour l'extraction d'ADN génomique à partir d'échantillons de palmiers dattiers, des instructions concernant les conditions d'amplification PCR, des instructions concernant l'analyse des amplicons obtenus, et/ou des instructions pour interpréter les résultats.

Un kit peut également comprendre une notice sous la forme prescrite par une agence gouvernementale régulant la préparation, la vente et l'utilisation de produits biologiques.

A moins qu'ils ne soient définis d'une autre manière, tous les termes techniques et scientifiques utilisés ici ont la même signification que celle couramment comprise par un spécialiste ordinaire du domaine auquel appartient cette invention.

### Exemple

L'exemple suivant décrit certains modes de réalisation de la présente invention. Cependant, il est entendu que l'exemple n'est présenté qu'à titre illustratif seulement et ne limite en aucun cas la portée de l'invention.

### Identification des Jeux de Marqueurs Moléculaires de type Microsatellite

### Matériels et Méthodes

*Matériel Végétal.* Une collection de 550 cultivars de *Phoenix dactylifera* collectés en Tunisie, en Algérie, au Maroc, au Pakistan, en Egypte et à Oman a été assemblée. Des échantillons de *P. canariensis, P. loureiroi, P. reclinata et P. roebelenii* ont également été collectés. Dans cet échantillon, une sous-collection de 100 variétés de référence a été sélectionnée sur la base de leur importance dans les principaux pays phoenicicoles.

*Extraction de l'ADN.* Chaque échantillon constitué de feuilles a été lyophilisé pendant 72 heures au moyen d'un lyophilisateur Alphal-4LD *Plus* (Fisher Scientific, France). Les feuilles lyophilisées ont été broyées avec le Tissu Lyser System (Qiagen, USA) puis l'extraction a été réalisée avec le kit Dneasy plant kit (Qiagen, USA) en suivant le protocole du fabriquant.

L'ADN obtenu a été dosé avec le spectrofluorimètre Tecan GENios™ (Tecan, Swisse). Les concentrations de tous les échantillons ont été ajustées à 10 ng/µL pour le reste de la manipulation.

*Analyses Génétiques.* Les analyses génétiques (nombre d'allèles, hétérozylotie observée et attendue, Fis, index de fixation Wright) ont été réalisées à l'aide du logiciel Genetix 4.05 (Belkhir et al., 2004, GENETIX 4.05, logiciel sous Windows TM pour la génétique des populations. Laboratoire Génome, Populations, Interactions, CNRS UMR 5171, Université de Montpellier II, Montpellier, France).

### Description des Marqueurs Microsatellites

Un nouvel ensemble optimisé de microsatellites a été défini par l'exploitation de marqueurs microsatellites décrits dans la littérature ou identifiés à partir des ressources génomiques disponibles.

Les marqueurs mPdCIR078, mPdCIR015, mPdCIR016, mPdCIR032, mPdCIR035, mPdCIR057, mPdCIR085, mPdCIR010, mPdCIR025, mPdCIR063 sont des loci dinucléotidiques (GA)n, qui ont été décrits par Billotte et al. (Molecular Ecology Notes, 2004, 4: 256-258).

Les marqueurs mPdIRD031, mPdIRD033, mPdIRD040 et mPdIRD013 sont des répétitions en tandem microsatellites de 3 ou 6 paires de bases que les présents inventeurs ont identifiées *in silico* dans des séquences codantes de la séquence du génome du palmier dattier (Al-Dous et al., Nature Biotechnology, 2011, 29: 521-527 - http://qatar-weill.cornell.edu/research/datepalmGenome/download.html). Ces 4 loci ont été sélectionnés parmi 47 pour leur capacité d'amplification et le polymorphisme qu'ils génèrent.

Les marqueurs PdCUC3-ssr1CUC et PdCUC3-ssr2 sont des séquences dinucléotidiques identifiées dans des gènes CUC (Adam et al., Molecular Biology and Evolution, 2010, 28: 1439-1454).

Les marqueurs PdAG1-ssr1 et PdAP3-ssr1 sont des séquences dinucléotidiques identifiées par les présents inventeurs respectivement dans les gènes AG1 et AP3 (non publié).

Le marqueur CpfM12 est un marqueur minisatellite du génome chloroplastique (Henderson et al., Conservation Genetics, 2006, 7: 213-223).

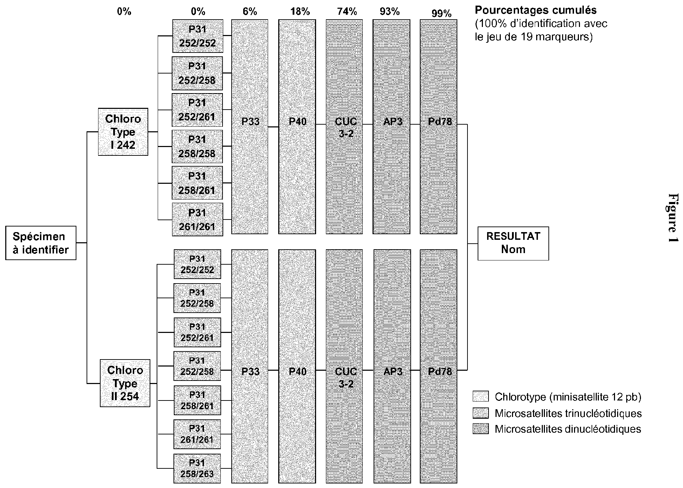

A partir de ces marqueurs, les inventeurs ont défini 2 jeux en fonction de l'objectif poursuivi : un jeu de 19 marqueurs qui permet d'identifier 100% des génotypes, un jeu réduit de 7 loci pour la certification des cultivars. Pour ce dernier, une clé d'identification a été définie qui permet d'identifier 99% des génotypes (Figure 1).

## Revendications

1. Méthode d'identification du génotype d'un palmier dattier par détection, à l'aide d'un jeu de marqueurs moléculaires mini-satellites et micro-satellites, d'un polymorphisme de type single sequence repeat (SSR) dans le génome du palmier dattier, **caractérisée en ce que** la détection du polymorphisme SSR comprend pour chaque marqueur moléculaire du jeu :
- l'amplification, à l'aide d'une paire d'amorces spécifiques du marqueur, d'une portion de l'ADN génomique du palmier dattier testé comprenant le mini-satellite ou micro-satellite pour obtenir des amplicons ; et
- l'analyse des amplicons obtenus pour déterminer le génotype du palmier dattier testé,
et **caractérisée en ce que** le jeu consiste en les 19 marqueurs moléculaires: mPdCIR010, mPdCIR015, mPdCIR016, mPdCIR025, mPdCIR032, mPdCIR035, mPdCIR057, mPdCIR063, mPdCIR078, mPdCIR085, CpfM12, PdCUC3-ssr1, PdCUC3-ssr2, PdAG1-ssr1, PdAP3-ssr, mPdIRD13, mPdIRD031, mPdIRD033, et mPdIRD40, ou **en ce que** le jeu consiste en les 7 marqueurs moléculaires: CpfM12, mPdIRD031, mPdIRD033, mPdIRD040, PdCUC3-ssr2, PdAP3-ssr, et mPdCIR078, où
- le marqueur mPdCIR010 a, dans le sens 5'→3', un motif (ga) ; est présent dans la séquence de numéro d'ordre GenBank AJ571673.1 ; et est flanqué, en 5', par la séquence nucléotidique SEQ ID NO: 1, et en 3', par la séquence nucléotidique SEQ ID NO: 2 ;
- le marqueur mPdCIR015 a, dans le sens 5'→3', un motif (ga) ; est présent dans la séquence de numéro d'ordre GenBank AJ571674.1 ; et est flanqué, en 5', par la séquence nucléotidique SEQ ID NO: 3, et en 3', par la séquence nucléotidique SEQ ID NO: 4 ;
- le marqueur mPdCIR016 a, dans le sens 5'→3', un motif (ga) ; est présent dans la séquence de numéro d'ordre GenBank AJ571675.1 ; et est flanqué, en 5', par la séquence nucléotidique SEQ ID NO: 5, et en 3', par la séquence nucléotidique SEQ ID NO: 6 ;
- le marqueur mPdCIR025 a, dans le sens 5'→3', un motif (ga) ; est présent dans la séquence de numéro d'ordre GenBank AJ571676.1 ; et est flanqué, en 5', par la séquence nucléotidique SEQ ID NO: 7, et en 3', par la séquence nucléotidique SEQ ID NO: 8 ;
- le marqueur mPdCIR032 a, dans le sens 5'→3', un motif (ga) ; est présent dans la séquence de numéro d'ordre GenBank AJ571677.1 ; et est flanqué, en 5', par la séquence nucléotidique SEQ ID NO: 9, et en 3', par la séquence nucléotidique SEQ ID NO: 10 ;
- le marqueur mPdCIR035 a, dans le sens 5'→3', un motif (ga) ; est présent dans la séquence de numéro d'ordre GenBank AJ571678.1 ; et est flanqué, en 5', par la séquence nucléotidique SEQ ID NO: 11, et en 3', par la séquence nucléotidique SEQ ID NO: 12 ;
- le marqueur mPdCIR057 a, dans le sens 5'→3', un motif (ga) ; est présent dans la séquence de numéro d'ordre GenBank AJ571682.1 ; et est flanqué, en 5', par la séquence nucléotidique SEQ ID NO: 13, et en 3', par la séquence nucléotidique SEQ ID NO: 14 ;
- le marqueur mPdCIR063 a, dans le sens 5'→3', un motif (ga) ; est présent dans la séquence de numéro d'ordre GenBank AJ571683.1 ; et est flanqué, en 5', par la séquence nucléotidique SEQ ID NO: 15, et en 3', par la séquence nucléotidique SEQ ID NO: 16 ;
- le marqueur mPdCIR078 a, dans le sens 5'→3', un motif (ga) ; est présent dans la séquence de numéro d'ordre GenBank AJ571685.1 ; et est flanqué, en 5', par la séquence nucléotidique SEQ ID NO: 17, et en 3', par la séquence nucléotidique SEQ ID NO: 18 ;
- le marqueur mPdCIR085 a, dans le sens 5'→3', un motif (ga) ; est présent dans la séquence de numéro d'ordre GenBank AJ571686.1 ; et est flanqué, en 5', par la séquence nucléotidique SEQ ID NO: 19, et en 3', par la séquence nucléotidique SEQ ID NO: 20 ;
- le marqueur CpfM12 a, dans le sens 5'→3', un motif (ctaactactata) ; est présent dans la séquence de numéro d'ordre GenBank EU043484.1 ; et est flanqué, en en 5', par la séquence nucléotidique SEQ ID NO: 21, et en 3', par la séquence nucléotidique SEQ ID NO: 22 ;
- le marqueur PdCUC3-ssr a, dans le sens 5'→3', un motif (gt+ga) ; est présent dans la séquence de numéro d'ordre GenBank HM622273.1 ; et est flanqué, en en 5', par la séquence nucléotidique SEQ ID NO: 23, et en 3', par la séquence nucléotidique SEQ ID NO: 24 ;
- le marqueur PdCUC3-ssr2 a, dans le sens 5'→3', un motif (ga) ; est présent dans la séquence de numéro d'ordre GenBank HM622273.1 ; et est flanqué, en en 5', par la séquence nucléotidique SEQ ID NO: 25, et en 3', par la séquence nucléotidique SEQ ID NO: 26 ;
- le marqueur PdAG1-ssr1 a, dans le sens 5'→3', un motif (ga) ; est présent dans la séquence de numéro d'ordre GenBank KC188338.1 ; et est flanqué, en en 5', par la séquence nucléotidique SEQ ID NO: 27, et en 3', par la séquence nucléotidique SEQ ID NO: 28 ;
- le marqueur PdAP3-ssr a, dans le sens 5'→3', un motif (ga) ; est présent dans la séquence de numéro d'ordre GenBank KC188337.1 ; et est flanqué, en en 5', par la séquence nucléotidique SEQ ID NO: 29, et en 3', par la séquence nucléotidique SEQ ID NO: 30 ;
- le marqueur mPdIRD13 a, dans le sens 5'→3', un motif (cac) ; est présent dans le scaffold PDK_20s1496731g002_1 de numéro d'ordre GenBank GL741615.1 ; et est flanqué, en en 5', par la séquence nucléotidique SEQ ID NO: 31, et en 3', par la séquence nucléotidique SEQ ID NO: 32 ;
- le marqueur mPdIRD31 a, dans le sens 5'→3', un motif (cca) ; est présent dans le scaffold PDK_20s1419261g003_1 ; et est flanqué, en en 5', par la séquence nucléotidique SEQ ID NO: 33, et en 3', par la séquence nucléotidique SEQ ID NO: 34 ;
- le marqueur mPdIRD33 a, dans le sens 5'→3', un motif (cag) ; est présent dans le scaffold PDK_20s1569281g001_1 ; et est flanqué, en en 5', par la séquence nucléotidique SEQ ID NO: 35, et en 3', par la séquence nucléotidique SEQ ID NO: 36 ; et
- le marqueur mPdIRD40 a, dans le sens 5'→3', un motif (ccagtg) ; est présent dans le scaffold PDK_20s1327401g002_1 ; et est flanqué, en 5', par la séquence nucléotidique SEQ ID NO: 37, et en 3', par la séquence nucléotidique SEQ ID NO: 38.

2. Méthode selon la revendication 1, **caractérisée en ce que** le jeu consiste en les 19 marqueurs moléculaires et les amplifications sont réalisées avec chacune des paires d'amorces spécifiques suivantes :
- une amorce SSR forward de séquence SEQ ID NO: 1 et une amorce SSR reverse de séquence SEQ ID NO: 2 ;
- une amorce SSR forward de séquence SEQ ID NO: 3 et une amorce SSR reverse de séquence SEQ ID NO: 4 ;
- une amorce SSR forward de séquence SEQ ID NO: 5 et une amorce SSR reverse de séquence SEQ ID NO: 6 ;
- une amorce SSR forward de séquence SEQ ID NO: 7 et une amorce SSR reverse de séquence SEQ ID NO: 8 ;
- une amorce SSR forward de séquence SEQ ID NO: 9 et une amorce SSR reverse de séquence SEQ ID NO: 10;
- une amorce SSR forward de séquence SEQ ID NO: 11 et une amorce SSR reverse de séquence SEQ ID NO: 12 ;
- une amorce SSR forward de séquence SEQ ID NO: 13 et une amorce SSR reverse de séquence SEQ ID NO: 14 ;
- une amorce SSR forward de séquence SEQ ID NO: 15 et une amorce SSR reverse de séquence SEQ ID NO: 16 ;
- une amorce SSR forward de séquence SEQ ID NO: 17 et une amorce SSR reverse de séquence SEQ ID NO: 18 ;
- une amorce SSR forward de séquence SEQ ID NO: 19 et une amorce SSR reverse de séquence SEQ ID NO: 20 ;
- une amorce SSR forward de séquence SEQ ID NO: 21 et une amorce SSR reverse de séquence SEQ ID NO: 22 ;
- une amorce SSR forward de séquence SEQ ID NO: 23 et une amorce SSR reverse de séquence SEQ ID NO: 24 ;
- une amorce SSR forward de séquence SEQ ID NO: 25 et une amorce SSR reverse de séquence SEQ ID NO: 26 ;
- une amorce SSR forward de séquence SEQ ID NO: 27 et une amorce SSR reverse de séquence SEQ ID NO: 28 ;
- une amorce SSR forward de séquence SEQ ID NO: 29 et une amorce SSR reverse de séquence SEQ ID NO: 30 ;
- une amorce SSR forward de séquence SEQ ID NO: 31 et une amorce SSR reverse de séquence SEQ ID NO: 32 ;
- une amorce SSR forward de séquence SEQ ID NO: 33 et une amorce SSR reverse de séquence SEQ ID NO: 34 ;
- une amorce SSR forward de séquence SEQ ID NO: 35 et une amorce SSR reverse de séquence SEQ ID NO: 36 ; et
- une amorce SSR forward de séquence SEQ ID NO: 37 et une amorce SSR reverse de séquence SEQ ID NO: 38.

3. Méthode selon la revendication 1, **caractérisée en ce que** le jeu consiste en les 7 marqueurs moléculaires et les amplifications sont réalisées avec chacune des paires d'amorces spécifiques suivantes :
- une amorce SSR forward de séquence SEQ ID NO: 17 et une amorce SSR reverse de séquence SEQ ID NO:18 ;
- une amorce SSR forward de séquence SEQ ID NO: 21 et une amorce SSR reverse de séquence SEQ ID NO:22 ;
- une amorce SSR forward de séquence SEQ ID NO: 25 et une amorce SSR reverse de séquence SEQ ID NO: 26 ;
- une amorce SSR forward de séquence SEQ ID NO: 29 et une amorce SSR reverse de séquence SEQ ID NO: 30 ;
- une amorce SSR forward de séquence SEQ ID NO: 33 et une amorce SSR reverse de séquence SEQ ID NO: 34 ;
- une amorce SSR forward de séquence SEQ ID NO: 35 et une amorce SSR reverse de séquence SEQ ID NO: 36 ; et
- une amorce SSR forward de séquence SEQ ID NO: 37 et une amorce SSR reverse de séquence SEQ ID NO: 38.

4. Méthode selon la revendication 1 ou la revendication 3, **caractérisée en ce que** le jeu consiste en les 7 marqueurs moléculaires et l'identification du génotype du palmier dattier testé comprend l'identification ou la certification du cultivar du palmier dattier testé.

5. Méthode selon l'une quelconque des revendications 1 à 4, **caractérisée en ce que** l'analyse des amplicons obtenus pour déterminer le génotype du palmier dattier testé comprend, pour chacun des marqueurs moléculaires :
- la séparation, en fonction de leur taille, des amplicons obtenus par amplification d'une portion de l'ADN génomique du palmier dattier testé, et
- la comparaison des tailles desdits amplicons avec les tailles des amplicons obtenus par amplification, dans les mêmes conditions, d'une portion de l'ADN génomique de palmiers dattiers contrôles.

6. Méthode selon l'une quelconque des revendications 1 à 5, **caractérisée en ce que** la méthode est réalisée sur de l'ADN génomique extrait d'un échantillon du palmier dattier testé, où l'échantillon du palmier dattier est un échantillon de protoplaste, de cal, d'embryons, de feuille, de stipe, de racine, de rejets ou de bouture du palmier dattier.

7. Jeu de paires d'amorces spécifiques de marqueurs moléculaires mini-satellites et micro-satellites pour identifier le génotype d'un palmier dattier, le jeu étant constitué des paires d'amorces suivantes :
- une amorce SSR forward de séquence SEQ ID NO: 1 et une amorce SSR reverse de séquence SEQ ID NO: 2 ;
- une amorce SSR forward de séquence SEQ ID NO: 3 et une amorce SSR reverse de séquence SEQ ID NO: 4 ;
- une amorce SSR forward de séquence SEQ ID NO: 5 et une amorce SSR reverse de séquence SEQ ID NO: 6 ;
- une amorce SSR forward de séquence SEQ ID NO: 7 et une amorce SSR reverse de séquence SEQ ID NO: 8 ;
- une amorce SSR forward de séquence SEQ ID NO: 9 et une amorce SSR reverse de séquence SEQ ID NO: 10;
- une amorce SSR forward de séquence SEQ ID NO: 11 et une amorce SSR reverse de séquence SEQ ID NO: 12 ;
- une amorce SSR forward de séquence SEQ ID NO: 13 et une amorce SSR reverse de séquence SEQ ID NO: 14 ;
- une amorce SSR forward de séquence SEQ ID NO: 15 et une amorce SSR reverse de séquence SEQ ID NO: 16 ;
- une amorce SSR forward de séquence SEQ ID NO: 17 et une amorce SSR reverse de séquence SEQ ID NO: 18 ;
- une amorce SSR forward de séquence SEQ ID NO: 19 et une amorce SSR reverse de séquence SEQ ID NO: 20 ;
- une amorce SSR forward de séquence SEQ ID NO: 21 et une amorce reverse de séquence SEQ ID NO: 22 ;
- une amorce SSR forward de séquence SEQ ID NO: 23 et une amorce SSR reverse de séquence SEQ ID NO: 24 ;
- une amorce SSR forward de séquence SEQ ID NO: 25 et une amorce SSR reverse de séquence SEQ ID NO: 26 ;
- une amorce SSR forward de séquence SEQ ID NO: 27 et une amorce SSR reverse de séquence SEQ ID NO: 28 ;
- une amorce SSR forward de séquence SEQ ID NO: 29 et une amorce SSR reverse de séquence SEQ ID NO: 30 ;
- une amorce SSR forward de séquence SEQ ID NO: 31 et une amorce SSR reverse de séquence SEQ ID NO: 32 ;
- une amorce SSR forward de séquence SEQ ID NO: 33 et une amorce SSR reverse de séquence SEQ ID NO: 34 ;
- une amorce SSR forward de séquence SEQ ID NO: 35 et une amorce SSR reverse de séquence SEQ ID NO: 36 ; et
- une amorce SSR forward de séquence SEQ ID NO: 37 et une amorce SSR reverse de séquence SEQ ID NO: 38.

8. Jeu de paires d'amorces spécifiques de marqueurs moléculaires mini-satellites et micro-satellites pour identifier ou certifier le cultivar d'un palmier dattier testé, le jeu étant constitué des paires d'amorces suivantes :
- une amorce SSR forward de séquence SEQ ID NO: 17 et une amorce SSR reverse de séquence SEQ ID NO:18 ;
- une amorce SSR forward de séquence SEQ ID NO: 21 et une amorce SSR reverse de séquence SEQ ID NO:22 ;
- une amorce SSR forward de séquence SEQ ID NO: 25 et une amorce SSR reverse de séquence SEQ ID NO: 26 ;
- une amorce SSR forward de séquence SEQ ID NO: 29 et une amorce SSR reverse de séquence SEQ ID NO: 30 ;
- une amorce SSR forward de séquence SEQ ID NO: 33 et une amorce SSR reverse de séquence SEQ ID NO: 34 ;
- une amorce SSR forward de séquence SEQ ID NO: 35 et une amorce SSR reverse de séquence SEQ ID NO: 36 ; et
- une amorce SSR forward de séquence SEQ ID NO: 37 et une amorce SSR reverse de séquence SEQ ID NO: 38.

9. Jeu de paires d'amorces selon la revendication 7 ou la revendication 8, **caractérisé en ce qu'**une des amorces de chacune des paires d'amorces comprend un marqueur détectable.

10. Utilisation du jeu de paires d'amorces selon la revendication 7 pour l'identification du génotype d'un palmier dattier.

11. Utilisation du jeu de paires d'amorces selon la revendication 8 pour l'identification ou la certification du cultivar d'un palmier dattier.

12. Kit pour l'identification du génotype de palmiers dattiers comprenant le jeu de paires d'amorces selon la revendication 7 et des instructions pour identifier le génotype par une méthode selon l'une quelconque des revendications 1, 2, 5 et 6 où le jeu consiste en les 19 marqueurs moléculaires.

13. Kit pour l'identification ou la certification du cultivar de palmiers dattiers comprenant le jeu de paires d'amorces selon la revendication 8 et des instructions pour identifier ou certifier le cultivar par une méthode selon l'une quelconque des revendications 1, 3, 4, 5 et 6 où le jeu consiste en les 7 marqueurs moléculaires.

## Patentansprüche

1. Verfahren zur Identifizierung des Genotyps einer Dattelpalme durch Detektion eines Polymorphismus des Typs Einzelsequenz-Repeat (SSR) im Dattelpalmengenom unter Verwendung eines Satzes von Minisatelliten- und Mikrosatelliten-Molekularmarkern, **dadurch gekennzeichnet, dass** die Detektion des SSR-Polymorphismus für jeden Molekularmarker des Satzes umfasst:
- Amplifikation, unter Verwendung eines Paars markerspezifischer Primer, eines Teils der genomischen DNA der getesteten Dattelpalme, umfassend den Minisatelliten oder Mikrosatelliten, um Amplikons zu erhalten; und
- Analyse der erhaltenen Amplikons, um den Genotyp der getesteten Dattelpalme zu bestimmen,
und **dadurch gekennzeichnet, dass** der Satz aus den 19 Molekularmarkern besteht: mPdCIR010, mPdCIR015, mPdCIR016, mPdCIR025, mPdCIR032, mPdCIR035, mPdCIR057, mPdCIR063, mPdCIR078, mPdCIR085, CpfM12, PdCUC3-ssr1, PdCUC3-ssr2, PdAG1-ssr1, PdAP3-ssr, mPdIRD13, mPdIRD031, mPdIRD033 und mPdIRD40, oder dass der Satz aus den 7 Molekularmarkern besteht: CpfM12, mPdIRD031, mPdIRD033, mPdIRD040, PdCUC3-ssr2, PdAP3-ssr und mPdCIR078, wobei
- der Marker mPdCIR010, in Richtung 5'→3', ein Motiv (ga) aufweist; in der Sequenz mit der GenBank Ordnungsnummer AJ571673.1 vorhanden ist; und am 5' von der Nukleotidsequenz SEQ ID NO: 1 und am 3' von der Nukleotidsequenz SEQ ID NO: 2 flankiert ist;
- der Marker mPdCIR015, in Richtung 5'→3', ein Motiv (ga) aufweist; in der Sequenz mit der GenBank Ordnungsnummer AJ571674.1 vorhanden ist; und am 5' von der Nukleotidsequenz SEQ ID NO: 3 und am 3' von der Nukleotidsequenz SEQ ID NO: 4 flankiert ist;
- der Marker mPdCIR016, in Richtung 5'→3', ein Motiv (ga) aufweist; in der Sequenz mit der GenBank Ordnungsnummer AJ571675.1 vorhanden ist; und am 5' von der Nukleotidsequenz SEQ ID NO: 5 und am 3' von der Nukleotidsequenz SEQ ID NO: 6 flankiert ist;
- der Marker mPdCIR025, in Richtung 5'→3', ein Motiv (ga) aufweist; in der Sequenz mit der GenBank Ordnungsnummer AJ571676.1 vorhanden ist; und am 5' von der Nukleotidsequenz SEQ ID NO: 7 und am 3' von der Nukleotidsequenz SEQ ID NO: 8 flankiert ist;
- der Marker mPdCIR032, in Richtung 5'→3', ein Motiv (ga) aufweist; in der Sequenz mit der GenBank Ordnungsnummer AJ571677.1 vorhanden ist; und am 5' von der Nukleotidsequenz SEQ ID NO: 9 und am 3' von der Nukleotidsequenz SEQ ID NO: 10 flankiert ist;
- der Marker mPdCIR035, in Richtung 5'→3', ein Motiv (ga) aufweist; in der Sequenz mit der GenBank Ordnungsnummer AJ571678.1 vorhanden ist; und am 5' von der Nukleotidsequenz SEQ ID NO: 11 und am 3' von der Nukleotidsequenz SEQ ID NO: 12 flankiert ist;
- der Marker mPdCIR057, in Richtung 5'→3', ein Motiv (ga) aufweist; in der Sequenz mit der GenBank Ordnungsnummer AJ571682.1 vorhanden ist; und am 5' von der Nukleotidsequenz SEQ ID NO: 13 und am 3' von der Nukleotidsequenz SEQ ID NO: 14 flankiert ist;
- der Marker mPdCIR063, in Richtung 5'→3', ein Motiv (ga) aufweist; in der Sequenz mit der GenBank Ordnungsnummer AJ571683.1 vorhanden ist; und am 5' von der Nukleotidsequenz SEQ ID NO: 15 und am 3' von der Nukleotidsequenz SEQ ID NO: 16 flankiert ist;
- der Marker mPdCIR078, in Richtung 5'→3', ein Motiv (ga) aufweist; in der Sequenz mit der GenBank Ordnungsnummer AJ571685.1 vorhanden ist; und am 5' von der Nukleotidsequenz SEQ ID NO: 17 und am 3' von der Nukleotidsequenz SEQ ID NO: 18 flankiert ist;
- der Marker mPdCIR085, in Richtung 5'→3', ein Motiv (ga) aufweist; in der Sequenz mit der GenBank Ordnungsnummer AJ571686.1 vorhanden ist; und am 5' von der Nukleotidsequenz SEQ ID NO: 19 und am 3' von der Nukleotidsequenz SEQ ID NO: 20 flankiert ist;
- der Marker CpfM12, in Richtung 5'→3', ein Motiv (ctaactactata) aufweist; in der Sequenz mit der GenBank Ordnungsnummer EU043484.1 vorhanden ist; und am 5' von der Nukleotidsequenz SEQ ID NO: 21 und am 3' von der Nukleotidsequenz SEQ ID NO: 22 flankiert ist;
- der Marker PdCUC3-ssr, in Richtung 5'→3', ein Motiv (gt+ga) aufweist; in der Sequenz mit der GenBank Ordnungsnummer HM622273.1 vorhanden ist; und am 5' von der Nukleotidsequenz SEQ ID NO: 23 und am 3' von der Nukleotidsequenz SEQ ID NO: 24 flankiert ist;
- der Marker PdCUC3-ssr2, in Richtung 5'→3', ein Motiv (ga) aufweist; in der Sequenz mit der GenBank Ordnungsnummer HM622273.1 vorhanden ist; und am 5' von der Nukleotidsequenz SEQ ID NO: 25 und am 3' von der Nukleotidsequenz SEQ ID NO: 26 flankiert ist;
- der Marker PdAG1-ssr1, in Richtung 5'→3', ein Motiv (ga) aufweist; in der Sequenz mit der GenBank Ordnungsnummer KC188338.1 vorhanden ist; und am 5' von der Nukleotidsequenz SEQ ID NO: 27 und am 3' von der Nukleotidsequenz SEQ ID NO: 28 flankiert ist;
- der Marker PdAP3-ssr, in Richtung 5'→3', ein Motiv (ga) aufweist; in der Sequenz mit der GenBank Ordnungsnummer KC188337.1 vorhanden ist; und am 5' von der Nukleotidsequenz SEQ ID NO: 29 und am 3' von der Nukleotidsequenz SEQ ID NO: 30 flankiert ist;
- der Marker mPdIRD13, in Richtung 5'→3', ein Motiv (cac) aufweist; in dem Scaffold PDK_20s1496731g002_1 mit der GenBank Ordnungsnummer GL741615.1 vorhanden ist; und am 5' von der Nukleotidsequenz SEQ ID NO: 31 und am 3' von der Nukleotidsequenz SEQ ID NO: 32 flankiert ist;
- der Marker mPdIRD31, in Richtung 5'→3', ein Motiv (cca) aufweist; in dem Scaffold PDK_20s1419261g003_1 vorhanden ist; und am 5' von der Nukleotidsequenz SEQ ID NO: 33 und am 3' von der Nukleotidsequenz SEQ ID NO: 34 flankiert ist;
- der Marker mPdIRD33, in Richtung 5'→3', ein Motiv (cag) aufweist; in dem Scaffold PDK_20s1569281g001_1 vorhanden ist; und am 5' von der Nukleotidsequenz SEQ ID NO: 35 und am 3' von der Nukleotidsequenz SEQ ID NO: 36 flankiert ist; und
- der Marker mPdIRD40, in Richtung 5'→3', ein Motiv (ccagtg) aufweist; in dem Scaffold PDK_20s1327401g002_1 vorhanden ist; und am 5' von der Nukleotidsequenz SEQ ID NO: 37 und am 3' von der Nukleotidsequenz SEQ ID NO: 38 flankiert ist.

2. Verfahren nach Anspruch 1, **dadurch gekennzeichnet, dass** der Satz aus den 19 Molekularmarkern besteht und die Amplifikationen mit jedem der folgenden spezifischen Primerpaare durchgeführt werden:
- einem Vorwärts-SSR-Primer der Sequenz SEQ ID NO: 1 und einem Rückwärts-SSR-Primer der Sequenz SEQ ID NO: 2;
- einem Vorwärts-SSR-Primer der Sequenz SEQ ID NO: 3 und einem Rückwärts-SSR-Primer der Sequenz SEQ ID NO: 4;
- einem Vorwärts-SSR-Primer der Sequenz SEQ ID NO: 5 und einem Rückwärts-SSR-Primer der Sequenz SEQ ID NO : 6;
- einem Vorwärts-SSR-Primer der Sequenz SEQ ID NO: 7 und einem Rückwärts-SSR-Primer der Sequenz SEQ ID NO: 8;
- einem Vorwärts-SSR-Primer der Sequenz SEQ ID NO: 9 und einem Rückwärts-SSR-Primer der Sequenz SEQ 10 NO: 10;
- einem Vorwärts-SSR-Primer der Sequenz SEQ ID NO: 11 und einem Rückwärts-SSR-Primer der Sequenz SEQ ID NO: 12;
- einem Vorwärts-SSR-Primer der Sequenz SEQ ID NO: 13 und einem Rückwärts-SSR-Primer der Sequenz SEQ ID NO: 14;
- einem Vorwärts-SSR-Primer der Sequenz SEQ ID NO: 15 und einem Rückwärts-SSR-Primer der Sequenz SEQ ID NO: 16;
- einem Vorwärts-SSR-Primer der Sequenz SEQ ID NO: 17 und einem Rückwärts-SSR-Primer der Sequenz SEQ ID NO: 18;
- einem Vorwärts-SSR-Primer der Sequenz SEQ ID NO: 19 und einem Rückwärts-SSR-Primer der Sequenz SEQ ID NO: 20;
- einem Vorwärts-SSR-Primer der Sequenz SEQ ID NO: 21 und einem Rückwärts-SSR-Primer der Sequenz SEQ ID NO: 22;
- einem Vorwärts-SSR-Primer der Sequenz SEQ ID NO: 23 und einem Rückwärts-SSR-Primer der Sequenz SEQ ID NO: 24;
- einem Vorwärts-SSR-Primer der Sequenz SEQ ID NO: 25 und einem Rückwärts-SSR-Primer der Sequenz SEQ ID NO: 26;
- einem Vorwärts-SSR-Primer der Sequenz SEQ ID NO: 27 und einem Rückwärts-SSR-Primer der Sequenz SEQ ID NO: 28;
- einem Vorwärts-SSR-Primer der Sequenz SEQ ID NO: 29 und einem Rückwärts-SSR-Primer der Sequenz SEQ ID NO: 30;
- einem Vorwärts-SSR-Primer der Sequenz SEQ ID NO: 31 und einem Rückwärts-SSR-Primer der Sequenz SEQ ID NO: 32;
- einem Vorwärts-SSR-Primer der Sequenz SEQ ID NO: 33 und einem Rückwärts-SSR-Primer der Sequenz SEQ ID NO: 34;
- einem Vorwärts-SSR-Primer der Sequenz SEQ ID NO: 35 und einem Rückwärts-SSR-Primer der Sequenz SEQ ID NO 36; und
- einem Vorwärts-SSR-Primer der Sequenz SEQ ID NO: 37 und einem Rückwärts-SSR-Primer der Sequenz SEQ ID NO: 38.

3. Verfahren nach Anspruch 1, **dadurch gekennzeichnet, dass** der Satz aus den 7 Molekularmarkern besteht und die Amplifikationen mit jedem der folgenden spezifischen Primerpaare durchgeführt werden:
- einem Vorwärts-SSR-Primer der Sequenz SEQ ID NO: 17 und einem Rückwärts-SSR-Primer der Sequenz SEQ ID NO: 18;
- einem Vorwärts-SSR-Primer der Sequenz SEQ ID NO: 21 und einem Rückwärts-SSR-Primer der Sequenz SEQ ID NO: 22;
- einem Vorwärts-SSR-Primer der Sequenz SEQ ID NO: 25 und einem Rückwärts-SSR-Primer der Sequenz SEQ ID NO: 26;
- einem Vorwärts-SSR-Primer der Sequenz SEQ ID NO: 29 und einem Rückwärts-SSR-Primer der Sequenz SEQ ID NO: 30;
- einem Vorwärts-SSR-Primer der Sequenz SEQ ID NO: 33 und einem Rückwärts-SSR-Primer der Sequenz SEQ ID NO: 34;
- einem Vorwärts-SSR-Primer der Sequenz SEQ ID NO: 35 und einem Rückwärts-SSR-Primer der Sequenz SEQ ID NO: 36; und
- einem Vorwärts-SSR-Primer der Sequenz SEQ ID NO: 37 und einem Rückwärts-SSR-Primer der Sequenz SEQ ID NO: 38.

4. Verfahren nach Anspruch 1 oder nach Anspruch 3, **dadurch gekennzeichnet, dass** der Satz aus den 7 Molekularmarkern besteht und die Identifizierung des Genotyps der getesteten Dattelpalme die Identifizierung oder die Zertifizierung der Sorte der getesteten Dattelpalme umfasst.

5. Verfahren nach einem der Ansprüche 1 bis 4, **dadurch gekennzeichnet, dass** die Analyse der erhaltenen Amplikons zum Bestimmen des Genotyps der getesteten Dattelpalme für jeden der Molekularmarker umfasst:
- die Trennung der durch Amplifikation eines Teils der genomischen DNA der getesteten Dattelpalme erhaltenen Amplikons, ihrer Größe nach, und
- den Vergleich der Größen dieser Amplikons mit den Größen der durch Amplifikation eines Teils der genomischen DNA der kontrollierten Dattelpalmen erhaltenen Amplikons unter den gleichen Bedingungen.

6. Verfahren nach einem der Ansprüche 1 bis 5, **dadurch gekennzeichnet, dass** das Verfahren an genomischer DNA durchgeführt wird, die aus einer Probe der getesteten Dattelpalme extrahiert ist, wobei die Probe der Dattelpalme eine Protoplastprobe, eine Kallusprobe, eine Probe aus Embryonen, eine Blattprobe, eine Stängelprobe, eine Wurzelprobe, eine Probe aus Sprossen oder eine Stecklingprobe der Dattelpalme ist.

7. Satz von Primerpaaren, die für Minisatelliten- und Mikrosatellitenmolekularmarker spezifisch sind, zum Identifizieren des Genotyps einer Dattelpalme, wobei der Satz aus den folgenden Primerpaaren besteht:
- einem Forward-SSR-Primer der Sequenz SEQ ID NO: 1 und einem Rückwärts-SSR-Primer der Sequenz SEQ ID NO: 2;
- einem Vorwärts-SSR-Primer der Sequenz SEQ ID NO: 3 und einem Rückwärts-SSR-Primer der Sequenz SEQ ID NO: 4;
- einem Vorwärts-SSR-Primer der Sequenz SEQ ID NO: 5 und einem Rückwärts-SSR-Primer der Sequenz SEQ ID NO: 6;
- einem Vorwärts-SSR-Primer der Sequenz SEQ ID NO: 7 und einem Rückwärts-SSR-Primer der Sequenz SEQ ID NO: 8;
- einem Vorwärts-SSR-Primer der Sequenz SEQ ID NO: 9 und einem Rückwärts-SSR-Primer der Sequenz SEQ ID NO: 10;
- einem Vorwärts-SSR-Primer der Sequenz SEQ ID NO: 11 und einem Rückwärts-SSR-Primer der Sequenz SEQ ID NO: 12;
- einem Vorwärts-SSR-Primer der Sequenz SEQ ID NO: 13 und einem Rückwärts-SSR-Primer der Sequenz SEQ ID NO: 14;
- einem Vorwärts-SSR-Primer der Sequenz SEQ ID NO: 15 und einem Rückwärts-SSR-Primer der Sequenz SEQ ID NO: 16;
- einem Vorwärts-SSR-Primer der Sequenz SEQ ID NO: 17 und einem Rückwärts-SSR-Primer der Sequenz SEQ ID NO 18;
- einem Vorwärts-SSR-Primer der Sequenz SEQ ID NO: 19 und einem Rückwärts-SSR-Primer der Sequenz SEQ ID NO: 20;
- einem Vorwärts-SSR-Primer der Sequenz SEQ ID NO: 21 und einem Rückwärts-Primer der Sequenz SEQ ID NO: 22;
- einem Vorwärts-SSR-Primer der Sequenz SEQ ID NO: 23 und einem Rückwärts-SSR-Primer der Sequenz SEQ ID NO: 24;
- einem Vorwärts-SSR-Primer der Sequenz SEQ ID NO: 25 und einem Rückwärts-SSR-Primer der Sequenz SEQ ID NO: 26;
- einem Vorwärts-SSR-Primer der Sequenz SEQ ID NO: 27 und einem Rückwärts-SSR-Primer der Sequenz SEQ ID NO: 28;
- einem Vorwärts-SSR-Primer der Sequenz SEQ ID NO: 29 und einem Rückwärts-SSR-Primer der Sequenz SEQ ID NO: 30;
- einem Vorwärts-SSR-Primer der Sequenz SEQ ID NO: 31 und einem Rückwärts-SSR-Primer der Sequenz SEQ ID NO: 32;
- einem Vorwärts-SSR-Primer der Sequenz SEQ ID NO: 33 und einem Rückwärts-SSR-Primer der Sequenz SEQ ID NO: 34;
- einem Vorwärts-SSR-Primer der Sequenz SEQ ID NO: 35 und einem Rückwärts-SSR-Primer der Sequenz SEQ ID NO: 36; und
- einem Vorwärts-SSR-Primer der Sequenz SEQ ID NO: 37 und einem Rückwärts-SSR-Primer der Sequenz SEQ ID NO: 38.

8. Satz von Primerpaaren, die für Minisatelliten- und Mikrosatellitenmolekularmarker spezifisch sind, zum Identifizieren oder Zertifizieren der Sorte einer getesteten Dattelpalme, wobei der Satz aus den folgenden Primerpaaren besteht:
- einem Vorwärts-SSR-Primer der Sequenz SEQ ID NO: 17 und einem Rückwärts-SSR-Primer der Sequenz SEQ ID NO: 18;
- einem Vorwärts-SSR-Primer der Sequenz SEQ ID NO: 21 und einem Rückwärts-SSR-Primer der Sequenz SEQ ID NO: 22;
- einem Vorwärts-SSR-Primer der Sequenz SEQ ID NO: 25 und einem Rückwärts-SSR-Primer der Sequenz SEQ ID NO: 26;
- einem Vorwärts-SSR-Primer der Sequenz SEQ ID NO: 29 und einem Rückwärts-SSR-Primer der Sequenz SEQ ID NO: 30;
- einem Vorwärts-SSR-Primer der Sequenz SEQ ID NO: 33 und einem Rückwärts-SSR-Primer der Sequenz SEQ ID NO: 34;
- einem Vorwärts-SSR-Primer der Sequenz SEQ ID NO: 35 und einem Rückwärts-SSR-Primer der Sequenz SEQ ID NO: 36; und
- einem Vorwärts-SSR-Primer der Sequenz SEQ ID NO: 37 und einem Rückwärts-SSR-Primer der Sequenz SEQ ID NO: 38.

9. Satz von Primerpaaren nach Anspruch 7 oder Anspruch 8, **dadurch gekennzeichnet, dass** einer der Primer von jedem der Primerpaare einen nachweisbaren Marker umfasst.

10. Verwendung des Satzes von Primerpaaren nach Anspruch 7 zur Identifizierung des Genotyps einer Dattelpalme.

11. Verwendung des Satzes von Primerpaaren nach Anspruch 8 zur Identifizierung oder Zertifizierung der Sorte einer Dattelpalme.

12. Kit zur Identifizierung des Genotyps von Dattelpalmen, umfassend den Satz von Primerpaaren nach Anspruch 7 und Anweisungen zum Identifizieren des Genotyps durch ein Verfahren nach einem der Ansprüche 1, 2, 5 und 6 wobei der Satz aus den 19 Molekularmarkern besteht.

13. Kit zur Identifizierung oder Zertifizierung der Sorte von Dattelpalmen, umfassend den Satz von Primerpaaren nach Anspruch 8 und Anweisungen zum Identifizieren oder Zertifizieren der Sorte durch ein Verfahren nach einem der Ansprüche 1, 3, 4, 5 und 6 wobei der Satz aus den 7 Molekularmarkern besteht.

## Claims

1. Method for identifying the genotype of a date palm by detecting, using a set of minisatellite and microsatellite molecular markers, a polymorphism of the single sequence repeat (SSR) type in the genome of the date palm, **characterized in that** the detecting of the SSR polymorphism comprises, for each molecular marker of the set:
- amplification, using a pair of primers specific for the marker, of a portion of the genomic DNA of the date palm tested comprising the minisatellite or microsatellite in order to obtain amplicons; and
- analysis of the amplicons obtained in order to determine the genotype of the date palm tested,
and **characterized in that** the set consists of the 19 molecular markers: mPdCIR010, mPdCIR015, mPdCIR016, mPdCIR025, mPdCIR032, mPdCIR035, mPdCIR057, mPdCIR063, mPdCIR078, mPdCIR085, CmpfM12, PdCUC3-ssr1, PdCUC3-ssr2, PdAG1-ssr1, PdAP3-ssr, mPdIRD13, mPdIRD031, mPdIRD033, and mPdIRD40, or **in that** the set consists of the 7 molecular markers: CpfM12, mPdIRD031, mPdIRD033, mPdIRD040, PdCUC3-ssr2, PdAP3-ssr-F4 and mPdCIR078, wherein:
- the mPdCIR010 marker has, in the 5'→3' direction, a (ga) motif; is present in the sequence having the GenBank accession number AJ571673.1, and is flanked, in 5', by the nucleotide sequence SEQ ID NO: 1 and, in 3', by the nucleotide sequence SEQ ID NO: 2;
- the mPdCIR015 marker has, in the 5'→3' direction, a (ga) motif; is present in the sequence having the GenBank accession number AJ571674.1, and is flanked, in 5', by the nucleotide sequence SEQ ID NO: 3 and, in 3', by the nucleotide sequence SEQ ID NO: 4;
- the mPdCIR016 marker has, in the 5'→3' direction, a (ga) motif; is present in the sequence having the GenBank accession number AJ571675.1, and is flanked, in 5', by the nucleotide sequence SEQ ID NO: 5 and, in 3', by the nucleotide sequence SEQ ID NO: 6;
- the mPdCIR025 marker has, in the 5'→3' direction, a (ga) motif; is present in the sequence having the GenBank accession number AJ571676.1, and is flanked, in 5', by the nucleotide sequence SEQ ID NO: 7 and, in 3', by the nucleotide sequence SEQ ID NO: 8;
- the mPdCIR032 marker has, in the 5'→3' direction, a (ga) motif; is present in the sequence having the GenBank accession number AJ571677.1, and is flanked, in 5', by the nucleotide sequence SEQ ID NO: 9 and, in 3', by the nucleotide sequence SEQ ID NO: 10;
- the mPdCIR035 marker has, in the 5'→3' direction, a (ga) motif; is present in the sequence having the GenBank accession number AJ571678.1, and is flanked, in 5', by the nucleotide sequence SEQ ID NO: 11 and, in 3', by the nucleotide sequence SEQ ID NO: 12;
- the mPdCIR057 marker has, in the 5'→3' direction, a (ga) motif; is present in the sequence having the GenBank accession number AJ571682.1, and is flanked, in 5', by the nucleotide sequence SEQ ID NO: 13 and, in 3', by the nucleotide sequence SEQ ID NO: 14;
- the mPdCIR063 marker has, in the 5'→3' direction, a (ga) motif; is present in the sequence having the GenBank accession number AJ571683.1, and is flanked, in 5', by the nucleotide sequence SEQ ID NO: 15 and, in 3', by the nucleotide sequence SEQ ID NO: 16;
- the mPdCIR078 marker has, in the 5'→3' direction, a (ga) motif; is present in the sequence having the GenBank accession number AJ571685.1, and is flanked, in 5', by the nucleotide sequence SEQ ID NO: 17 and, in 3', by the nucleotide sequence SEQ ID NO: 18;
- the mPdCIR085 marker has, in the 5'→3' direction, a (ga) motif; is present in the sequence having the GenBank accession number AJ571686.1, and is flanked, in 5', by the nucleotide sequence SEQ ID NO: 19 and, in 3', by the nucleotide sequence SEQ ID NO: 20;
- the CpfM12 marker has, in the 5'→3' direction, a (ctaactactata) motif; is present in the sequence having the GenBank accession number EU043484.1, and is flanked, in 5', by the nucleotide sequence SEQ ID NO: 21 and, in 3', by the nucleotide sequence SEQ ID NO: 22;
- the PdCUC3-ssr marker has, in the 5'→3' direction, a (gt+ga) motif; is present in the sequence having the GenBank accession number HM622273.1, and is flanked, in 5', by the nucleotide sequence SEQ ID NO: 23 and, in 3', by the nucleotide sequence SEQ ID NO: 24;
- the PdCUC3-ssr2 marker has, in the 5'→3' direction, a (ga) motif; is present in the sequence having the GenBank accession number HM622273.1, and is flanked, in 5', by the nucleotide sequence SEQ ID NO: 25 and, in 3', by the nucleotide sequence SEQ ID NO: 26;
- the PdAG1-ssr1 marker has, in the 5'→3' direction, a (ga) motif; is present in the sequence having the GenBank accession number KC188338.1, and is flanked, in 5', by the nucleotide sequence SEQ ID NO: 27 and, in 3', by the nucleotide sequence SEQ ID NO: 28;
- the PdAP3-ssr marker has, in the 5'→3' direction, a (ga) motif; is present in the sequence having the GenBank accession number KC188337.1, and is flanked, in 5', by the nucleotide sequence SEQ ID NO: 29 and, in 3', by the nucleotide sequence SEQ ID NO: 30;
- the mPdIRD13 marker has, in the 5'→3' direction, a (cac) motif; is present in the PDK_20s1496731g002_1 scaffold having the GenBank accession number GL741615.1, and is flanked, in 5', by the nucleotide sequence SEQ ID NO: 31 and, in 3', by the nucleotide sequence SEQ ID NO: 32;
- the mPdIRD31 marker has, in the 5'→3' direction, a (cca) motif; is present in the PDK_20s1419261g003_1 scaffold, and is flanked, in 5', by the nucleotide sequence SEQ ID NO: 33 and, in 3', by the nucleotide sequence SEQ ID NO: 34;
- the mPdIRD33 marker has, in the 5'→3' direction, a (cag) motif; is present in the PDK_20s1569281g001_1 scaffold, and is flanked, in 5', by the nucleotide sequence SEQ ID NO: 35 and, in 3', by the nucleotide sequence SEQ ID NO: 36; and
- the mPdIRD40 marker has, in the 5'→3' direction, a (ccagtg) motif; is present in the PDK_20s1327401g002_1 scaffold, and is flanked, in 5', by the nucleotide sequence SEQ ID NO: 37 and, in 3', by the nucleotide sequence SEQ ID NO: 38.

2. Method according to Claim 1, **characterized in that** the set consists of the 19 molecular markers and the amplifications are carried out with each of the following pairs of specific primers:
- a forward SSR primer of sequence SEQ ID NO: 1 and a reverse SSR primer of sequence SEQ ID NO: 2;
- a forward SSR primer of sequence SEQ ID NO: 3 and a reverse SSR primer of sequence SEQ ID NO: 4;
- a forward SSR primer of sequence SEQ ID NO: 5 and a reverse SSR primer of sequence SEQ ID NO: 6;
- a forward SSR primer of sequence SEQ ID NO: 7 and a reverse SSR primer of sequence SEQ ID NO: 8;
- a forward SSR primer of sequence SEQ ID NO: 9 and a reverse SSR primer of sequence SEQ ID NO: 10;
- a forward SSR primer of sequence SEQ ID NO: 11 and a reverse SSR primer of sequence SEQ ID NO: 12;
- a forward SSR primer of sequence SEQ ID NO: 13 and a reverse SSR primer of sequence SEQ ID NO: 14;
- a forward SSR primer of sequence SEQ ID NO: 15 and a reverse SSR primer of sequence SEQ ID NO: 16;
- a forward SSR primer of sequence SEQ ID NO: 17 and a reverse SSR primer of sequence SEQ ID NO: 18;
- a forward SSR primer of sequence SEQ ID NO: 19 and a reverse SSR primer of sequence SEQ ID NO: 20;
- a forward SSR primer of sequence SEQ ID NO: 21 and a reverse SSR primer of sequence SEQ ID NO: 22;
- a forward SSR primer of sequence SEQ ID NO: 23 and a reverse SSR primer of sequence SEQ ID NO: 24;
- a forward SSR primer of sequence SEQ ID NO: 25 and a reverse SSR primer of sequence SEQ ID NO: 26;
- a forward SSR primer of sequence SEQ ID NO: 27 and a reverse SSR primer of sequence SEQ ID NO: 28;
- a forward SSR primer of sequence SEQ ID NO: 29 and a reverse SSR primer of sequence SEQ ID NO: 30;
- a forward SSR primer of sequence SEQ ID NO: 31 and a reverse SSR primer of sequence SEQ ID NO: 32;
- a forward SSR primer of sequence SEQ ID NO: 33 and a reverse SSR primer of sequence SEQ ID NO: 34;
- a forward SSR primer of sequence SEQ ID NO: 35 and a reverse SSR primer of sequence SEQ ID NO: 36; and
- a forward SSR primer of sequence SEQ ID NO: 37 and a reverse SSR primer of sequence SEQ ID NO: 38.

3. Method according to Claim 1, **characterized in that** the set consists of the 7 molecular markers and the amplifications are carried out with each of the following pairs of specific primers:
- a forward SSR primer of sequence SEQ ID NO: 17 and a reverse SSR primer of sequence SEQ ID NO: 18;
- a forward SSR primer of sequence SEQ ID NO: 21 and a reverse SSR primer of sequence SEQ ID NO: 22;
- a forward SSR primer of sequence SEQ ID NO: 25 and a reverse SSR primer of sequence SEQ ID NO: 26;
- a forward SSR primer of sequence SEQ ID NO: 29 and a reverse SSR primer of sequence SEQ ID NO: 30;
- a forward SSR primer of sequence SEQ ID NO: 33 and a reverse SSR primer of sequence SEQ ID NO: 34;
- a forward SSR primer of sequence SEQ ID NO: 35 and a reverse SSR primer of sequence SEQ ID NO: 36; and
- a forward SSR primer of sequence SEQ ID NO: 37 and a reverse SSR primer of sequence SEQ ID NO: 38.

4. Method according to Claim 1 or Claim 3, **characterized in that** the set consists of the 7 molecular markers and the identification of the genotype of the date palm tested comprises the identification or the certification of the cultivar of the date palm tested.

5. Method according to any one of Claims 1 to 4, **characterized in that** the analysis of the amplicons obtained in order to determine the genotype of the date palm tested comprises, for each of the molecular markers:
- separation, as a function of their size, of the amplicons obtained by amplification of a portion of the genomic DNA of the date palm tested, and
- comparison of the sizes of said amplicons with the sizes of the amplicons obtained by amplification, under the same conditions, of a portion of the genomic DNA of control date palms.

6. Method according to any one of Claims 1 to 5, **characterized in that** the method is carried out on genomic DNA extracted from a sample of the date palm tested, where the sample of the date palm is a protoplast, callus, embryo, leaf, stipe, root, shoot or cutting sample of the date palm.

7. Set of pairs of primers specific for minisatellite and microsatellite molecular markers for identifying the genotype of a date palm, the set consisting of the following pairs of primers:
- a forward SSR primer of sequence SEQ ID NO: 1 and a reverse SSR primer of sequence SEQ ID NO: 2;
- a forward SSR primer of sequence SEQ ID NO: 3 and a reverse SSR primer of sequence SEQ ID NO: 4;
- a forward SSR primer of sequence SEQ ID NO: 5 and a reverse SSR primer of sequence SEQ ID NO: 6;
- a forward SSR primer of sequence SEQ ID NO: 7 and a reverse SSR primer of sequence SEQ ID NO: 8;
- a forward SSR primer of sequence SEQ ID NO: 9 and a reverse SSR primer of sequence SEQ ID NO: 10;
- a forward SSR primer of sequence SEQ ID NO: 11 and a reverse SSR primer of sequence SEQ ID NO: 12;
- a forward SSR primer of sequence SEQ ID NO: 13 and a reverse SSR primer of sequence SEQ ID NO: 14;
- a forward SSR primer of sequence SEQ ID NO: 15 and a reverse SSR primer of sequence SEQ ID NO: 16;
- a forward SSR primer of sequence SEQ ID NO: 17 and a reverse SSR primer of sequence SEQ ID NO: 18;
- a forward SSR primer of sequence SEQ ID NO: 19 and a reverse SSR primer of sequence SEQ ID NO: 20;
- a forward SSR primer of sequence SEQ ID NO: 21 and a reverse primer of sequence SEQ ID NO: 22;
- a forward SSR primer of sequence SEQ ID NO: 23 and a reverse SSR primer of sequence SEQ ID NO: 24;
- a forward SSR primer of sequence SEQ ID NO: 25 and a reverse SSR primer of sequence SEQ ID NO: 26;
- a forward SSR primer of sequence SEQ ID NO: 27 and a reverse SSR primer of sequence SEQ ID NO: 28;
- a forward SSR primer of sequence SEQ ID NO: 29 and a reverse SSR primer of sequence SEQ ID NO: 30;
- a forward SSR primer of sequence SEQ ID NO: 31 and a reverse SSR primer of sequence SEQ ID NO: 32;
- a forward SSR primer of sequence SEQ ID NO: 33 and a reverse SSR primer of sequence SEQ ID NO: 34;
- a forward SSR primer of sequence SEQ ID NO: 35 and a reverse SSR primer of sequence SEQ ID NO: 36; and
- a forward SSR primer of sequence SEQ ID NO: 37 and a reverse SSR primer of sequence SEQ ID NO: 38.

8. Set of pairs of primers specific for minisatellite and microsatellite molecular markers for identifying or certifying the cultivar of a date palm tested, the set consisting of the following pairs of primers:
- a forward SSR primer of sequence SEQ ID NO: 17 and a reverse SSR primer of sequence SEQ ID NO: 18;
- a forward SSR primer of sequence SEQ ID NO: 21 and a reverse SSR primer of sequence SEQ ID NO: 22;
- a forward SSR primer of sequence SEQ ID NO: 25 and a reverse SSR primer of sequence SEQ ID NO: 26;
- a forward SSR primer of sequence SEQ ID NO: 29 and a reverse SSR primer of sequence SEQ ID NO: 30;
- a forward SSR primer of sequence SEQ ID NO: 33 and a reverse SSR primer of sequence SEQ ID NO: 34;
- a forward SSR primer of sequence SEQ ID NO: 35 and a reverse SSR primer of sequence SEQ ID NO: 36; and
- a forward SSR primer of sequence SEQ ID NO: 37 and a reverse SSR primer of sequence SEQ ID NO: 38.

9. Set of pairs of primers according to Claim 7 or Claim 8, **characterized in that** one of the primers of each of the pairs of primers comprises a detectable marker.

10. Use of the set of pairs of primers according to Claim 7 for identifying the genotype of a date palm.

11. Use of the set of pairs of primers according to Claim 8 for identifying or certifying the cultivar of a date palm.

12. Kit for identifying the genotype of date palms, comprising the set of pairs of primers according to Claim 7 and instructions for identifying the genotype using a method according to any one of Claims 1, 2, 5 and 6 where the set consists of the 19 molecular markers.

13. Kit for identifying or certifying the cultivar of date palms, comprising the set of pairs of primers according to Claim 8 and instructions for identifying or certifying the cultivar using a method according to any one of Claims 1, 3, 4, 5 and 6 where the set consists of the 7 molecular markers.


### SEQUENCE LISTING

<110> IRD
<120> Marqueurs Moléculaires et Méthodes pour l'Identification des Génotypes de Palmier Dattier
<130> BCT130364 QT
<150> FR1261226
   <151> 2012-11-26
<160> 38
<170> PatentIn version 3.5
<210> 1
   <211> 17
   <212> DNA
   <213> Artificial Sequence
<220>
   <223> amorce SSR forward pour marqueur mPdCIR010
<400> 1
   accccggacg tgaggtg 17
<210> 2
   <211> 22
   <212> DNA
   <213> Artificial Sequence
<220>
   <223> amorce SSR reverse pour marqueur mPdCIR010
<400> 2
   cgtcgatctc ctcctttgtc tc 22
<210> 3
   <211> 20
   <212> DNA
   <213> Artificial Sequence
<220>
   <223> amorce SSR forward pour marqueur mPdCIR015
<400> 3
   agctggctcc tcccttctta 20
<210> 4
   <211> 19
   <212> DNA
   <213> Artificial Sequence
<220>
   <223> amorce SSR reverse pour marqueur mPdCIR015
<400> 4
   gctcggttgg acttgttct 19
<210> 5
   <211> 20
   <212> DNA
   <213> Artificial Sequence
<220>
   <223> amorce SSR forward pour marqueur mPdCIR016
<400> 5
   agcgggaaat gaaaaggtat 20
<210> 6
   <211> 20
   <212> DNA
   <213> Artificial Sequence
<220>
   <223> amorce SSR reverse pour marqueur mPdCIR016
<400> 6
   atgaaaacgt gccaaatgtc 20
<210> 7
   <211> 19
   <212> DNA
   <213> Artificial Sequence
<220>
   <223> amorce SSR forward pour marqueur mPdCIR025
<400> 7
   gcacgagaag gcttatagt 19
<210> 8
   <211> 19
   <212> DNA
   <213> Artificial Sequence
<220>
   <223> amorce SSR reverse pour marqueur mPdCIR025
<400> 8
   cccctcatta ggattctac 19
<210> 9
   <211> 17
   <212> DNA
   <213> Artificial Sequence
<220>
   <223> amorce SSR forward pour marqueur mPdCIR032
<400> 9
   caaatctttg ccgtgag 17
<210> 10
   <211> 23
   <212> DNA
   <213> Artificial Sequence
<220>
   <223> amorce SSR reverse pour marqueur mPdCIR032
<400> 10
   ggtgtggagt aatcatgtag tag 23
<210> 11
   <211> 20
   <212> DNA
   <213> Artificial Sequence
<220>
   <223> amorce SSR forward pour marqueur mPdCIR035
<400> 11
   acaaacggcg atgggattac 20
<210> 12
   <211> 20
   <212> DNA
   <213> Artificial Sequence
<220>
   <223> amorce SSR reverse pour marqueur mPdCIR035
<400> 12
   ccgcagctca cctcttctat 20
<210> 13
   <211> 20
   <212> DNA
   <213> Artificial Sequence
<220>
   <223> amorce SSR forward pour marqueur mPdCIR057
<400> 13
   aagcagcagc ccttccgtag 20
<210> 14
   <211> 22
   <212> DNA
   <213> Artificial Sequence
<220>
   <223> amorce SSR reverse pour marqueur mPdCIR057
<400> 14
   gttctcactc gcccaaaaat ac 22
<210> 15
   <211> 20
   <212> DNA
   <213> Artificial Sequence
<220>
   <223> amorce SSR forward pour marqueur mPdCIR063
<400> 15
   cttttatgtg gtctgagaga 20
<210> 16
   <211> 20
   <212> DNA
   <213> Artificial Sequence
<220>
   <223> amorce SSR reverse pour marqueur mPdCIR063
<400> 16
   tctctgatct tgggttctgt 20
<210> 17
   <211> 17
   <212> DNA
   <213> Artificial Sequence
<220>
   <223> amorce SSR forward pour marqueur mPdCIR078
<400> 17
   tggatttcca ttgtgag 17
<210> 18
   <211> 17
   <212> DNA
   <213> Artificial Sequence
<220>
   <223> amorce SSR reverse pour marqueur mPdCIR078
<400> 18
   cccgaagaga cgctatt 17
<210> 19
   <211> 19
   <212> DNA
   <213> Artificial Sequence
<220>
   <223> amorce SSR forward pour marqueur mPdCIR085
<400> 19
   gagagagggt ggtgttatt 19
<210> 20
   <211> 19
   <212> DNA
   <213> Artificial Sequence
<220>
   <223> amorce SSR reverse pour marqueur mPdCIR085
<400> 20
   ttcatccaga accacagta 19
<210> 21
   <211> 22
   <212> DNA
   <213> Artificial Sequence
<220>
   <223> amorce SSR forward pour marqueur CpfM12
<400> 21
   ccgcccacga tgaagtaatg ta 22
<210> 22
   <211> 23
   <212> DNA
   <213> Artificial Sequence
<220>
   <223> amorce SSR reverse pour marqueur CpfM12
<400> 22
   gtcacgggtt caaatcctgt ctc 23
<210> 23
   <211> 24
   <212> DNA
   <213> Artificial Sequence
<220>
   <223> amorce SSR forward pour marqueur PdCUC3-ssr1
<400> 23
   cgtggactca tgactcgcat gtcc 24
<210> 24
   <211> 20
   <212> DNA
   <213> Artificial Sequence
<220>
   <223> amorce SSR reverse pour marqueur PdCUC3-ssr1
<400> 24
   ggtccttgcc ggtggccttc 20
<210> 25
   <211> 23
   <212> DNA
   <213> Artificial Sequence
<220>
   <223> amorce SSR forward pour marqueur PdCUC3-ssr2
<400> 25
   acattgctct tttgccatgg gct 23
<210> 26
   <211> 20
   <212> DNA
   <213> Artificial Sequence
<220>
   <223> amorce SSR reverse pour marqueur PdCUC3-ssr2
<400> 26
   cgagcaggtg gggttcgggt 20
<210> 27
   <211> 24
   <212> DNA
   <213> Artificial Sequence
<220>
   <223> amorce SSR forward pour marqueur PdAG1-ssr1
<400> 27
   tctgatttcg tttacttctt agga 24
<210> 28
   <211> 23
   <212> DNA
   <213> Artificial Sequence
<220>
   <223> amorce SSR reverse pour marqueur PdAG1-ssr1
<400> 28
   ttcatattca gttgtcgggt gta 23
<210> 29
   <211> 22
   <212> DNA
   <213> Artificial Sequence
<220>
   <223> amorce SSR forward pour marqueur PdAP3-ssr
<400> 29
   gagaaataga gagctgtgca ag 22
<210> 30
   <211> 21
   <212> DNA
   <213> Artificial Sequence
<220>
   <223> amorce SSR reverse pour marqueur PdAP3-ssr
<400> 30
   ctgcagtact cggagaactt g 21
<210> 31
   <211> 20
   <212> DNA
   <213> Artificial Sequence
<220>
   <223> amorce SSR forward pour marqueur mPdIRD13
<400> 31
   gcggagacag gagatggtaa 20
<210> 32
   <211> 20
   <212> DNA
   <213> Artificial Sequence
<220>
   <223> amorce SSR reverse pour marqueur mPdIRD13
<400> 32
   cttgactgct tctgctgctg 20
<210> 33
   <211> 20
   <212> DNA
   <213> Artificial Sequence
<220>
   <223> amorce SSR forward pour marqueur mPdIRD31
<400> 33
   gcaggtggac tgcaaaatct 20
<210> 34
   <211> 20
   <212> DNA
   <213> Artificial Sequence
<220>
   <223> amorce SSR reverse pour marqueur mPdIRD31
<400> 34
   ctattggggt gctgatccat 20
<210> 35
   <211> 20
   <212> DNA
   <213> Artificial Sequence
<220>
   <223> amorce SSR forward pour marqueur mPdIRD33
<400> 35
   ggagcataca gtgggtttgc 20
<210> 36
   <211> 20
   <212> DNA
   <213> Artificial Sequence
<220>
   <223> amorce SSR reverse pour marqueur mPdIRD33
<400> 36
   cagcctggga atgaggatag 20
<210> 37
   <211> 20
   <212> DNA
   <213> Artificial Sequence
<220>
   <223> amorce SSR forward pour marqueur mPdIRD40
<400> 37
   gagagatgcg tcagggaatc 20
<210> 38
   <211> 20
   <212> DNA
   <213> Artificial Sequence
<220>
   <223> amorce SSR reverse pour marqueur mPdIRD40
<400> 38
   ccagaatctt ccaagcaagc 20